(19) 

**Europäisches Patentamt**
**European Patent Office**
**Office européen des brevets**

(11) **EP 4 722 189 A1**

(12) **EUROPEAN PATENT APPLICATION**
published in accordance with Art. 153(4) EPC

(43) Date of publication:
08.04.2026  Bulletin 2026/15

(21) Application number: 24815381.9

(22) Date of filing: 23.05.2024

(51) International Patent Classification (IPC):
*C07C 69/30* (2006.01)  *A61K 8/86* (2006.01)
*A61Q 5/00* (2006.01)  *A61Q 19/00* (2006.01)
*C07B 61/00* (2006.01)  *C07C 67/26* (2006.01)
*C07C 69/58* (2006.01)  *C08G 65/22* (2006.01)
*C11C 3/00* (2006.01)

(52) Cooperative Patent Classification (CPC):
A61K 8/39; A61K 8/86; A61Q 1/00; A61Q 5/00;
A61Q 19/00; C07B 61/00; C07C 67/08;
C07C 67/26; C07C 69/30; C07C 69/33;
C07C 69/58; C08G 65/22; C11C 3/00; C11D 3/2003

(86) International application number:
PCT/JP2024/019098

(87) International publication number:
WO 2024/247897 (05.12.2024 Gazette 2024/49)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 26.05.2023  JP 2023087300

(71) Applicant: **Daicel Corporation**
**Osaka-shi, Osaka 530-0011 (JP)**

(72) Inventors:
• **NAKAMURA, Ryouta**
**Tokyo 108-8230 (JP)**

• **SATO, Tomohiko**
**Tokyo 108-8230 (JP)**
• **SUZUKI, Youji**
**Tokyo 108-8230 (JP)**
• **KAGEYUKI, Hitomi**
**Tokyo 108-8230 (JP)**
• **SAKAMOTO, Yuta**
**Tokyo 108-8230 (JP)**
• **OKAUCHI, Shunnosuke**
**Tokyo 108-8230 (JP)**

(74) Representative: **Grünecker Patent- und
Rechtsanwälte
PartG mbB
Leopoldstraße 4
80802 München (DE)**

(54) **POLYGLYCERIN MONO-FATTY ACID ESTER PRODUCT AND METHOD FOR PRODUCING SAME**

(57)  Provided is a polyglycerol mono-fatty acid ester product with high purity. In addition, there is provided a production method capable of providing a polyglycerol mono-fatty acid ester with high purity. A polyglycerol mono-fatty acid ester product, in which a fatty acid has 4 to 25 carbon atoms, a peak intensity ratio represented by Formula (X) below is 0.20 or more, and a peak intensity ratio represented by Formula (Y) below is 0.46 or less: Formula (X) = P2/P1 Formula (Y) = P3/P1 P1: a sum of peak intensities of a monoester form, a dehydration product of the monoester form, a diester form, a dehydration product of the diester form, a triester form, and a dehydration product of the triester form of a polyglycerol fatty acid ester, as well as peak intensities of polyglycerol and a dehydration product of the polyglycerol when mass spectrometry of the polyglycerol mono-fatty acid ester product is performed using a time-of-flight mass spectrometer, P2: the peak intensity of the monoester form of the polyglycerol fatty acid ester when the mass spectrometry of the polyglycerol mono-fatty acid ester product is performed using the time-of-flight mass spectrometer, P3: a sum of the peak intensities of the polyglycerol and the dehydration product of the polyglycerol when the mass spectrometry of the polyglycerol mono-fatty acid ester product is performed using the time-of-flight mass spectrometer.

FIG. 1

## Description

Technical Field

The present disclosure relates to a polyglycerol mono-fatty acid ester product and a method for producing the same.

Background Art

Polyglycerol mono-fatty acid esters exhibit various properties by the combination of the degree of polymerization of polyglycerol and fatty acids having different chain lengths. They have been widely used as surfactants in applications such as food additives, detergent compositions, and cosmetics (cosmetic compositions). Examples of a method for producing such a polyglycerol fatty acid ester include an esterification reaction of polyglycerol with a fatty acid, a transesterification reaction of polyglycerol with a fatty acid ester, and an addition polymerization reaction of glycidol with a fatty acid (Patent Documents 1 to 6).

In addition, Patent Document 7 describes a polyglycerol monolaurate form obtained by reacting lauric acid/-glycidol/phosphoric acid while supplying them to a reactor at a specific ratio. Furthermore, Patent Document 8 describes an invention of a polyglycerol monooleate composition obtained by charging a linear diglycerol monooleate to a flask and reacting it with glycidol and phosphoric acid that are being added dropwise.

However, the polyglycerol mono-fatty acid esters known so far contain a large amount of by-products other than a monoester form (e.g., a dehydration product of the monoester form, a diester form, a dehydration product of the diester form, a triester form, and a dehydration product of the triester form), and it cannot be said that the purity of the monoester form is high. Therefore, when the polyglycerol mono-fatty acid ester is used as a surfactant, there is a problem that the surface tension cannot be efficiently reduced.

Citation List

Patent Document

Patent Document 1: JP 8-109153 A
Patent Document 2: JP 9-117258 A
Patent Document 3: JP 9-216813 A
Patent Document 4: JP 9-272893 A
Patent Document 5: JP 2006-111539 A
Patent Document 6: WO 2004/048304
Patent Document 7: JP 2001-139679 A
Patent Document 8: JP 2017-071586 A

Summary of Invention

Technical Problem

The polyglycerol mono-fatty acid ester product of Patent Document 7 is obtained by a method for continuously supplying and reacting a fatty acid, an acidic catalyst, and glycidol, and thus it does not satisfy the ranges of the peak intensity ratios represented by Formula (X) and Formula (Y) in the present disclosure described later. In addition, the polyglycerol mono-fatty acid ester product of Patent Document 8 uses a diglycerol mono-fatty acid ester as a raw material instead of a fatty acid, and thus it does not satisfy the ranges of the peak intensity ratios represented by Formula (X) and Formula (Y) in the present disclosure described later.

Accordingly, an object of the present disclosure is to provide a polyglycerol mono-fatty acid ester product with high purity. Another object of the present disclosure is to provide a production method capable of producing a polyglycerol mono-fatty acid ester with high purity.

Solution to Problem

The inventors of the present disclosure have conducted intensive studies to solve the above problems, and as a result, have found a polyglycerol mono-fatty acid ester product with high purity. The invention according to the present disclosure has been accomplished based on these findings.

That is, the present disclosure provides a polyglycerol mono-fatty acid ester product, in which:

a fatty acid has from 4 to 25 carbon atoms;
a peak intensity ratio represented by Formula (X) below is 0.20 or more; and
a peak intensity ratio represented by Formula (Y) below is 0.46 or less.

$$\text{Formula } (X) = P2/P1$$

$$\text{Formula } (Y) = P3/P1$$

P1: a sum of peak intensities of a monoester form, a dehydration product of the monoester form, a diester form, a dehydration product of the diester form, a triester form, and a dehydration product of the triester form of a polyglycerol fatty acid ester, as well as peak intensities of polyglycerol and a dehydration product of the polyglycerol, when mass spectrometry of the polyglycerol mono-fatty acid ester product is performed using a time-of-flight mass spectrometer

P2: the peak intensity of the monoester form of the polyglycerol fatty acid ester when the mass spectrometry of the polyglycerol mono-fatty acid ester product is performed using the time-of-flight mass spectrometer

P3: a sum of the peak intensities of the polyglycerol and the dehydration product of the polyglycerol when the mass spectrometry of the polyglycerol mono-fatty acid ester product is performed using the time-of-flight mass spectrometer

Preferably, in the polyglycerol mono-fatty acid ester product, the fatty acid has from 15 to 25 carbon atoms.

Preferably, in the polyglycerol mono-fatty acid ester product, the fatty acid has from 4 to 14 carbon atoms.

Preferably, in the polyglycerol mono-fatty acid ester product, the fatty acid has from 16 to 18 carbon atoms.

Preferably, in the polyglycerol mono-fatty acid ester product, the fatty acid has from 8 to 14 carbon atoms.

Preferably, in the polyglycerol mono-fatty acid ester product, an average degree of polymerization of glycerol is from 2 to 20.

Preferably, in the polyglycerol mono-fatty acid ester product, the fatty acid is a linear fatty acid.

Preferably, in the polyglycerol mono-fatty acid ester product, the fatty acid is an unsaturated fatty acid.

Preferably, in the polyglycerol mono-fatty acid ester product, the fatty acid is a branched fatty acid.

Preferably, the polyglycerol mono-fatty acid ester product is a reaction product of glycidol and a fatty acid.

The present disclosure also provides a cosmetic composition containing the polyglycerol mono-fatty acid ester product described above.

The present disclosure also provides a method for producing a polyglycerol mono-fatty acid ester product, the method including carrying out an addition polymerization reaction of glycidol with a fatty acid.

Preferably, in the method for producing the polyglycerol mono-fatty acid ester product, the addition polymerization reaction is carried out by continuously or intermittently supplying glycidol to the fatty acid.

Preferably, in the method for producing the polyglycerol mono-fatty acid ester product, the addition polymerization reaction is carried out by, while continuously or intermittently supplying glycidol to the fatty acid, continuously or intermittently supplying an acidic catalyst thereto.

Preferably, the acidic catalyst is a phosphoric acid or an acidic phosphate ester.

Advantageous Effects of Invention

The polyglycerol mono-fatty acid ester product of the present disclosure has the above constitution, and thus contains a polyglycerol mono-fatty acid ester with high purity. In addition, according to the production method of the present disclosure, a polyglycerol mono-fatty acid ester product with high purity can be obtained.

Brief Description of Drawings

FIG. 1 is a spectrum of HPLC analysis of polyglycerol monocaprate of Example 1.
FIG. 2 is a chart of the corrected molecular weight and the corresponding peak intensity of the polyglycerol monocaprate of Example 1. The hollow bar indicates the monoester form.

Description of Embodiments

[Polyglycerol Mono-fatty Acid Ester Product]

The polyglycerol mono-fatty acid ester product of the present disclosure is characterized in that a fatty acid has from 4 to 25 carbon atoms, a peak intensity ratio represented by Formula (X) below is 0.20 or more, and a peak intensity ratio represented by Formula (Y) below is 0.46 or less.

$$\text{Formula (X)} = P2/P1$$

$$\text{Formula (Y)} = P3/P1$$

P1: a sum of peak intensities of a monoester form, a dehydration product of the monoester form, a diester form, a dehydration product of the diester form, a triester form, and a dehydration product of the triester form of a polyglycerol fatty acid ester, as well as peak intensities of polyglycerol and a dehydration product of the polyglycerol, when mass spectrometry of the polyglycerol mono-fatty acid ester product is performed using a time-of-flight mass spectrometer
P2: the peak intensity of the monoester form of the polyglycerol fatty acid ester when the mass spectrometry of the polyglycerol mono-fatty acid ester product is performed using the time-of-flight mass spectrometer
P3: a sum of the peak intensities of the polyglycerol and the dehydration product of the polyglycerol when the mass spectrometry of the polyglycerol mono-fatty acid ester product is performed using the time-of-flight mass spectrometer

The polyglycerol mono-fatty acid ester product of the present disclosure contains at least a polyglycerol mono-fatty acid ester. In addition to the polyglycerol mono-fatty acid ester, a polyglycerol poly-fatty acid ester, such as a polyglycerol di-fatty acid ester or a polyglycerol tri-fatty acid ester, may be contained. In addition, polyglycerol and the like, which are raw materials of the polyglycerol mono-fatty acid ester product, may also be contained. Furthermore, dehydration products of these described later may also be contained.
The polyglycerol mono-fatty acid ester is represented by, for example, Formula (1) below.

$$RC(=O)\text{-}[C_3H_6O_2]_n\text{-}OH \qquad \dots (1)$$

In Formula (1), R represents an aliphatic hydrocarbon group having from 3 to 24 carbon atoms. Examples of the aliphatic hydrocarbon group include an alkyl group, an alkenyl group, or an alkyl group having a hydroxy group. The number of carbon atoms of the aliphatic hydrocarbon group corresponds to that obtained by subtracting 1 from "the number of carbon atoms of the fatty acid" described below. n is the average degree of polymerization of glycerol, and represents, for example, from 2 to 20.
$C_3H_6O_2$ in the parentheses of Formula (1) above includes at least one structure selected from the group consisting of Formula (2), Formula (3), and Formula (4) below.

$$\text{-O-CH}_2\text{-CH(OH)-CH}_2\text{-} \qquad \dots (2)$$

$$\text{-O-CH(CH}_2\text{OH)-CH}_2\text{-} \qquad \dots (3)$$

$$\text{-O-CH}_2\text{-CH(CH}_2\text{OH)-} \qquad \dots (4)$$

In the present specification, the polyglycerol mono-fatty acid ester may simply be referred to as a "monoester form". The polyglycerol di-fatty acid ester is a compound in which two hydroxyl groups of polyglycerol are bound to carboxyl groups of fatty acids by forming ester bonds. Such a polyglycerol mono-fatty acid ester may simply be referred to as a "diester form". The polyglycerol tri-fatty acid ester is a compound in which three hydroxyl groups of polyglycerol are bound to carboxyl groups of fatty acids by forming ester bonds. Such a polyglycerol mono-fatty acid ester may simply be referred to as a "triester form". In addition, a polyglycerol poly-fatty acid ester other than the polyglycerol di-fatty acid ester and the polyglycerol tri-fatty acid ester may be referred to as a "tetraester form", a "pentaester form", a "hexaester form", or the like in the same manner as described above. These polyglycerol poly-fatty acid esters may be referred to as "polyester forms".
In the polyglycerol mono-fatty acid ester product of the present disclosure, a part of the polyglycerol may be dehydrated in the production process. In the polyglycerol mono-fatty acid ester, a product obtained with a part of the polyglycerol dehydrated may be referred to as a dehydration product of the polyglycerol mono-fatty acid ester, a dehydration product of the monoester form of the polyglycerol fatty acid ester, or simply a dehydration product of the monoester form. Similarly, a polyglycerol poly-fatty acid ester, such as a polyglycerol di-fatty acid ester or a polyglycerol tri-fatty acid ester, may be referred to as a dehydration product of the polyglycerol poly-fatty acid ester or a dehydration product of the polyester form, such as a dehydration product of the polyglycerol di-fatty acid ester or a dehydration product of the diester form, or a dehydration product of the polyglycerol tri-fatty acid ester or a dehydration product of the triester form. These dehydration products may have a cyclic structure.

The polyglycerol mono-fatty acid ester of the present disclosure may be obtained by (1) a reaction in which glycidol is subjected to addition polymerization with a fatty acid, (2) an esterification reaction of polyglycerol with a fatty acid, or (3) a transesterification reaction of polyglycerol with a fatty acid ester. The polyglycerol mono-fatty acid ester obtained by the above reaction (1) can alternatively be referred to as a reaction product of glycidol and a fatty acid. The polyglycerol mono-fatty acid ester obtained by the above reaction (2) can alternatively be referred to as an esterified form of polyglycerol and a fatty acid. The polyglycerol mono-fatty acid ester obtained by the above reaction (3) can alternatively be referred to as a transesterified form of polyglycerol and a fatty acid ester. Among these, the reaction product of glycidol and a fatty acid by the reaction (1) is preferable in that the proportion of the monoester form in the polyglycerol mono-fatty acid ester product becomes high.

(Time-of-Flight Mass Spectrometer (TOF-MS))

The time-of-flight mass spectrometer (TOF-MS) and the measurement conditions thereof for the polyglycerol mono-fatty acid ester product of the present disclosure are not particularly limited, and the measurement can be performed, for example, with the following apparatus and conditions.

- Apparatus: Xevo G2-XSQTof (Waters Corporation)
- Injection method: Injection by infusion (5 μL/min)
- Measurement mode: Neg
- Cone gas: 50 L/h
- Desolvation gas: 1000 L/h
- Source temp: 120°C
- Desolvation temp: 500°C
- Capillary: 2.0 kV
- Sampling cone: 40
- Source offset:80
- Sample concentration: 25 ppm (methanol)
- MS range: from 50 to 3000

In the present disclosure, the peak intensity ratio represented by Formula (X) is calculated by P2/P1. The peak intensity ratio represented by Formula (Y) is calculated by P3/P1.

The P1 is the sum of the peak intensities of a monoester form, a dehydration product of the monoester form, a diester form, a dehydration product of the diester form, a triester form, and a dehydration product of the triester form of a polyglycerol fatty acid ester, as well as the peak intensities of polyglycerol and a dehydration product of the polyglycerol, when the mass spectrometry of the polyglycerol mono-fatty acid ester product is performed using the time-of-flight mass spectrometer.

The P2 is the peak intensity of the monoester form of the polyglycerol fatty acid ester when the mass spectrometry of the polyglycerol mono-fatty acid ester product is performed using the time-of-flight mass spectrometer.

The P3 is the sum of the peak intensities of the polyglycerol and the dehydration product of the polyglycerol when the mass spectrometry of the polyglycerol mono-fatty acid ester product is performed using the time-of-flight mass spectrometer.

The peak intensities of the respective components used in the P1 to 3 are derived from the peak intensities obtained when the mass spectrometry of the polyglycerol mono-fatty acid ester product is performed using the time-of-flight mass spectrometer.

When the mass spectrometry of the polyglycerol mono-fatty acid ester product is performed using the time-of-flight mass spectrometer, a peak corresponding to the molecular weight of the monoester form (i.e., polyglycerol mono-fatty acid ester) as the polyglycerol fatty acid ester, as well as peaks corresponding to the molecular weights of the dehydration product of the monoester form, diester form, dehydration product of the diester form, triester form, dehydration product of the triester form, and the like, as impurities, can be observed. In addition, peaks corresponding to the molecular weights of a tetraester form, a dehydration product of the tetraester form, a pentaester form, a dehydration product of the pentaester form, and the like as polyglycerol poly-fatty acid esters and dehydration products thereof other than those described above, as impurities, can be observed. Furthermore, peaks corresponding to the molecular weights of the raw materials of the polyglycerol mono-fatty acid ester product, as well as the molecular weights of polyglycerol, a dehydration product of the polyglycerol, and the like as by-products, all of which being impurities, can be observed.

The polyglycerol mono-fatty acid ester and the impurities can be identified by the molecular weights measured when the mass spectrometry of the polyglycerol mono-fatty acid ester product is performed using the time-of-flight mass spectrometer.

Hereinafter, outlines of methods for calculating the above Formula (X), Formula (Y), and P1 to 3 will be described. Note that the specific methods will be described in Examples.

(a) Mass spectrometry of the polyglycerol mono-fatty acid ester product is performed using a time-of-flight mass spectrometer to obtain the molecular weight derived from each of the components described in P1 to 3 and the peak intensity thereof, and the numerical value of each molecular weight is rounded down to an integer by discarding the fractional portion, and the numerical values of the corresponding peak intensities are summed. The above-described rounding down of the molecular weight to an integer is intended to make it correspond to the corrected molecular weight described in (c) below.

(b) The theoretical molecular weight of each of the components described in P1 to 3 is calculated.

(c) The theoretical molecular weight in (b) is corrected in consideration of the following (i) to (iv), and the corrected molecular weight is defined.

(i) The molecular weight measured and detected by the time-of-flight mass spectrometer is derived from a molecule deprotonated by ionization. (ii) Regarding (i), the molecular weight for the peak top of the intensity may be further shifted by 1 for a molecule having a large molecular weight. (iii) Since some of the components described in P1 to 3 have the same molecular weight, it is necessary to appropriately select and calculate the molecular weight. (iv) In a case where a degree of dehydration cannot exist in consideration of the esterification number and the degree of polymerization of glycerol of the polyglycerol fatty acid ester, it is necessary to eliminate the degree of dehydration.

(d) The intensities obtained in (a) are made to correspond to the corrected molecular weights of the respective components of the polyglycerol mono-fatty acid ester product calculated in (c), and these are added up to obtain the peak intensities of the "monoester form", "dehydration product of monoester form", "diester form", "dehydration product of diester form", "triester form", "dehydration product of triester form", "polyglycerol", and "dehydration product of polyglycerol" described in P1 to 3. The peak intensity of each of the above components was calculated as follows.

- The monoester form, diester form, triester form, and polyglycerol are each obtained by summing the peak intensities in the case where the degree of polymerization of glycerol is from 1 to 20.
- The dehydration product of the monoester form, the dehydration product of the diester form, the dehydration product of the triester form, and the dehydration product of the polyglycerol are each obtained by summing the peak intensities in the case where the degree of polymerization of glycerol is from 1 to 20 and in the case where the degree of dehydration is from 1 to 3 (1 to 3 water molecules are removed).

(e) Formula (X) and Formula (Y) above are calculated using the peak intensities of the respective components of the polyglycerol mono-fatty acid ester product calculated in (d).

The P1 is the sum of the peak intensities of a monoester form, a dehydration product of the monoester form, a diester form, a dehydration product of the diester form, a triester form, and a dehydration product of the triester form of a polyglycerol fatty acid ester, as well as the peak intensities of polyglycerol and a dehydration product of the polyglycerol, when the mass spectrometry of the polyglycerol mono-fatty acid ester product is performed using the time-of-flight mass spectrometer.

The P2 is the peak intensity of the polyglycerol mono-fatty acid ester when the mass spectrometry of the polyglycerol mono-fatty acid ester product is performed using the time-of-flight mass spectrometer. Therefore, the peak intensity ratio represented by Formula (X) = P2/P1 can be said to be an indicator of the content of the polyglycerol mono-fatty acid ester (monoester form of the polyglycerol fatty acid ester) contained in the polyglycerol mono-fatty acid ester product. That is, a large value of Formula (X) means that the purity of the polyglycerol mono-fatty acid ester in the polyglycerol mono-fatty acid ester product is high.

The P3 is the sum of the peak intensities of the polyglycerol and the dehydration product of the polyglycerol when the mass spectrometry of the polyglycerol mono-fatty acid ester product is performed using the time-of-flight mass spectrometer. Therefore, the peak intensity ratio represented by Formula (Y) = P3/P1 can be said to be an indicator of the content of the polyglycerol and the dehydration product of the polyglycerol contained in the polyglycerol mono-fatty acid ester product. That is, a small value of Formula (Y) means that the content of the polyglycerol and the dehydration product of the polyglycerol in the polyglycerol mono-fatty acid ester product is small, and means that the content of the monoester form in the polyglycerol fatty acid ester is relatively large (purity is high).

Since the polyglycerol mono-fatty acid ester product of the present disclosure has a high purity, a surface tension can be efficiently reduced when used as a surfactant. In addition, the detergency and the ability to form liquid crystals (e.g., bicontinuous cubic liquid crystals) are good. Furthermore, when used as a cleansing oil, it exhibits good blendability in makeup, good makeup removability, and a good refreshing feeling on the skin after washing off makeup. This is considered to be an effect due to the formation of clean micelles because of the high purity.

The peak intensity ratio represented by Formula (X) above is not particularly limited as long as it is 0.20 or more, and it is more preferably 0.21 or more, more preferably 0.23 or more, more preferably 0.24 or more, more preferably 0.25 or more, more preferably 0.26 or more, more preferably 0.28 or more, more preferably 0.3 or more, more preferably 0.31 or more, more preferably 0.33 or more, more preferably 0.35 or more, more preferably 0.37 or more, more preferably 0.40 or more, more preferably 0.44 or more, more preferably 0.48 or more, and more preferably 0.52 or more. The peak intensity ratio represented by Formula (X) above is not particularly limited, and is, for example, 1 or less, preferably 0.9 or less, more preferably 0.8 or less, more preferably 0.75 or less, more preferably 0.7 or less, more preferably 0.65 or less, and more preferably 0.6 or less.

The peak intensity ratio represented by Formula (Y) above is not particularly limited as long as it is 0.46 or less, and is preferably 0.45 or less, more preferably 0.44 or less, more preferably 0.42 or less, more preferably 0.39 or less, more preferably 0.37 or less, more preferably 0.35 or less, more preferably 0.33 or less, more preferably 0.32 or less, and more preferably 0.30 or less. The peak intensity ratio represented by Formula (Y) above is not particularly limited, and is, for example, 0.01 or more, preferably 0.05 or more, and more preferably 0.10 or more.

In the polyglycerol mono-fatty acid ester product of the present disclosure, the average degree of polymerization of glycerol is not particularly limited, and is preferably from 2 to 40. The average degree of polymerization of glycerol may be 3 or more, 4 or more, 5 or more, 6 or more, 7 or more, 8 or more, or 9 or more. In addition, the average degree of polymerization of glycerol may be 35 or less, 30 or less, 25 or less, 20 or less, 18 or less, 16 or less, 14 or less, 12 or less, 10 or less, 8 or less, 6 or less, or 4 or less. The average degree of polymerization of glycerol may also be from 3 to 20, or from 4 to 10.

In the polyglycerol mono-fatty acid ester product, the method for specifying the average degree of polymerization of glycerol is not particularly limited, and for example, in the case of the polyglycerol mono-fatty acid ester product obtained by the reaction (1), it can be specified by the number of moles of glycidol to be subjected to addition polymerization per mole of the fatty acid. For example, the average degree of polymerization of glycerol of n means that, when n moles of glycidol are subjected to addition polymerization per mole of the fatty acid, the average degree of polymerization of glycerol of the resulting polyglycerol mono-fatty acid ester product is n. In addition, for example, in the case of the polyglycerol mono-fatty acid ester product obtained by the reactions (2) and (3), it is the same as the average degree of polymerization of glycerol of the polyglycerol to be used. For example, the polyglycerol mono-fatty acid ester product, obtained by an esterification reaction of the polyglycerol having an average degree of polymerization of glycerol of m with the fatty acid, has an average degree of polymerization of glycerol of m.

Another method for specifying the average degree of polymerization of glycerol is a method using a hydroxyl value. The hydroxyl value refers to the number of mg of potassium hydroxide required to neutralize the acetic acid bound to a hydroxyl group when 1 g of a sample is acetylated (see JIS K 0070-1992). The amount of hydroxyl groups in the polyglycerol chain of the polyglycerol mono-fatty acid ester product is calculated from the hydroxyl value, whereby its average degree of polymerization can be determined.

In the polyglycerol mono-fatty acid ester product of the present disclosure, the hydroxyl value is not particularly limited, and is preferably from 317 to 754 KOH/g. The hydroxyl value of the polyglycerol mono-fatty acid ester product may be 317 KOH/g or more, 389 KOH/g or more, 413 KOH/g or more, 475 KOH/g or more, 483 KOH/g or more, or 539 KOH/g or more. The hydroxyl value of the polyglycerol mono-fatty acid ester product may be 754 KOH/g or less, 751 KOH/g or less, 745 KOH/g or less, 741 KOH/g or less, 734 KOH/g or less, 725 KOH/g or less, 713 KOH/g or less, 698 KOH/g or less, or 677 KOH/g or less.

In the polyglycerol mono-fatty acid ester product of the present disclosure, the fatty acid is not particularly limited, and is preferably a fatty acid having, for example, from 4 to 25 carbon atoms. The fatty acid may be a saturated fatty acid or an unsaturated fatty acid. In addition, the fatty acid may be a linear fatty acid or a branched fatty acid (fatty acid having a side chain). Furthermore, the fatty acid may be a substituted fatty acid in which a part of hydrogen atoms bound to the carbon chain is substituted with a hydroxy group. Here, the fatty acid means a fatty acid as a constituent component of the polyglycerol mono-fatty acid ester.

The number of carbon atoms of the fatty acid is not particularly limited, and may be 6 or more, 8 or more, 10 or more, 12 or more, 14 or more, 15 or more, 16 or more, or 18 or more. The number of carbon atoms of the fatty acid is not particularly limited, and may be 22 or less, 20 or less, 18 or less, 16 or less, 14 or less, 12 or less, 10 or less, or 8 or less. The number of carbon atoms of the fatty acid may be 4 to 14 or 15 to 25, or may be 8 to 14 or 16 to 18.

The fatty acid is not particularly limited, and examples thereof include caproic acid ($C_6$, saturated fatty acid),

caprylic acid ($C_8$, saturated fatty acid), pelargonic acid ($C_9$, saturated fatty acid), 2-ethylhexanoic acid ($C_8$, branched fatty acid), capric acid ($C_{10}$, saturated fatty acid), lauric acid ($C_{12}$, saturated fatty acid), isotridecanoic acid ($C_{13}$, branched fatty acid), myristic acid ($C_{14}$, saturated fatty acid), pentadecanoic acid ($C_{15}$, saturated fatty acid), palmitic acid ($C_{16}$, saturated fatty acid), palmitoleic acid ($C_{16}$, unsaturated fatty acid), stearic acid ($C_{18}$, saturated fatty acid), isostearic acid ($C_{18}$, branched fatty acid), oleic acid ($C_{18}$, unsaturated fatty acid), linoleic acid ($C_{18}$, unsaturated fatty acid), ricinoleic acid ($C_{18}$, unsaturated fatty acid), hydroxystearic acid ($C_{18}$, substituted fatty acid), arachidic acid ($C_{20}$, saturated fatty acid), behenic acid ($C_{22}$, saturated fatty acid), erucic acid ($C_{22}$, unsaturated fatty acid), and nervonic acid ($C_{24}$, unsaturated fatty acid).

When the number of carbon atoms of the fatty acid is from 4 to 14 (particularly from 8 to 14), the peak intensity ratio represented by Formula (X) above is not particularly limited as long as it is 0.20 or more, and is more preferably 0.23 or more, more preferably 0.25 or more, more preferably 0.30 or more, more preferably 0.33 or more, more preferably 0.35 or more, and more preferably 0.40 or more.

When the number of carbon atoms of the fatty acid is from 4 to 14 (particularly from 8 to 14), the peak intensity ratio represented by Formula (Y) above is not particularly limited as long as it is 0.46 or less, and is preferably 0.45 or less, more preferably 0.42 or less, more preferably 0.39 or less, and more preferably 0.37 or less. The peak intensity ratio represented by Formula (Y) above is not particularly limited, and is, for example, 0.01 or more, preferably 0.05 or more, and more preferably 0.10 or more.

When the number of carbon atoms of the fatty acid is from 4 to 14 (particularly from 8 to 14), the average degree of polymerization of glycerol is not particularly limited, and is preferably from 2 to 40, more preferably from 3 to 20, still more preferably from 4 to 10, and particularly preferably from 6 to 10.

When the number of carbon atoms of the fatty acid is from 15 to 25 (particularly from 16 to 18), the peak intensity ratio represented by Formula (X) above is not particularly limited as long as it is 0.20 or more, and is more preferably 0.23 or more, more preferably 0.24 or more, more preferably 0.25 or more, more preferably 0.28 or more, more preferably 0.30 or more, more preferably 0.31 or more, more preferably 0.33 or more, and more preferably 0.35 or more.

When the number of carbon atoms of the fatty acid is from 15 to 25 (particularly from 16 to 18), the peak intensity ratio represented by Formula (Y) above is not particularly limited as long as it is 0.46 or less, and is preferably 0.44 or less, more preferably 0.37 or less, more preferably 0.32 or less, more preferably 0.30 or less, and more preferably 0.28 or less. The peak intensity ratio represented by Formula (Y) above is not particularly limited, and is, for example, 0.01 or more, preferably 0.05 or more, and more preferably 0.10 or more.

When the number of carbon atoms of the fatty acid is from 15 to 25 (particularly from 16 to 18), the average degree of polymerization of glycerol is not particularly limited, and is preferably from 2 to 40, more preferably from 3 to 20, and still more preferably from 4 to 10.

When the fatty acid is a linear fatty acid having from 15 to 25 (particularly from 16 to 18) carbon atoms, the peak intensity ratio represented by Formula (X) above is not particularly limited as long as it is 0.20 or more, but is more preferably 0.24 or more, more preferably 0.28 or more, and more preferably 0.30 or more. In this case, the average degree of polymerization of glycerol is not particularly limited, and is preferably from 2 to 40, more preferably from 3 to 20, and still more preferably from 4 to 10.

When the fatty acid is a linear fatty acid having from 15 to 25 (particularly from 16 to 18) carbon atoms, the average degree of polymerization of glycerol is not particularly limited, and is preferably from 2 to 40, more preferably from 3 to 20, and still more preferably from 4 to 10.

When the fatty acid is a branched fatty acid having from 15 to 25 (particularly from 16 to 18) carbon atoms, the peak intensity ratio represented by Formula (X) above is not particularly limited as long as it is 0.20 or more, and is preferably 0.25 or more, more preferably 0.30 or more, and more preferably 0.33 or more. In this case, the average degree of polymerization of glycerol is not particularly limited, and is preferably from 2 to 40, more preferably from 3 to 20, and still more preferably from 4 to 10.

When the fatty acid is a branched fatty acid having from 15 to 25 (particularly from 16 to 18) carbon atoms, the peak intensity ratio represented by Formula (Y) above is not particularly limited as long as it is 0.46 or less, and is preferably 0.40 or less, more preferably 0.37 or less, and more preferably 0.35 or less. The peak intensity ratio represented by Formula (Y) above is not particularly limited, and is, for example, 0.01 or more, preferably 0.05 or more, and more preferably 0.10 or more.

When the fatty acid is a branched fatty acid having from 15 to 25 (particularly from 16 to 18) carbon atoms, the average degree of polymerization of glycerol is not particularly limited, and is preferably from 2 to 40, more preferably from 3 to 20, and still more preferably from 4 to 10.

When the fatty acid is an unsaturated fatty acid having from 15 to 25 (particularly from 16 to 18) carbon atoms, the peak intensity ratio represented by Formula (X) above is not particularly limited as long as it is 0.20 or more, but is more preferably 0.23 or more, more preferably 0.25 or more, more preferably 0.30 or more, and more preferably 0.33 or more. In this case, the average degree of polymerization of glycerol is not particularly limited, and is preferably from 2 to 40, more preferably from 3 to 20, and still more preferably from 4 to 10.

When the fatty acid is an unsaturated fatty acid having from 15 to 25 (particularly from 16 to 18) carbon atoms, the peak intensity ratio represented by Formula (Y) above is not particularly limited as long as it is 0.46 or less, and is preferably 0.44 or less, more preferably 0.32 or less, more preferably 0.30 or less, and more preferably 0.28 or less. The peak intensity ratio represented by Formula (Y) above is not particularly limited, and is, for example, 0.01 or more, preferably 0.05 or more, and more preferably 0.10 or more.

When the fatty acid is an unsaturated fatty acid having from 15 to 25 (particularly from 16 to 18) carbon atoms, the average degree of polymerization of glycerol is not particularly limited, and is preferably from 2 to 40, more preferably from 3 to 20, and still more preferably from 4 to 10.

In the polyglycerol mono-fatty acid ester product of the present disclosure and in the following high performance liquid chromatography (HPLC) analysis, the area ratio of the peaks corresponding to the monoester form, dehydration product of the monoester form, polyester form, and dehydration product of the polyester form of the polyglycerol fatty acid ester, as well as the peaks corresponding to the polyglycerol and the dehydration product of the polyglycerol, is not particularly limited, and is preferably, for example, 50% or more, more preferably 60% or more, still more preferably 80% or more, and particularly preferably 90% or more.

In the polyglycerol mono-fatty acid ester product of the present disclosure and in the following high performance liquid chromatography (HPLC) analysis, the area ratio of the peaks corresponding to the monoester form, dehydration product of the monoester form, polyester form, and dehydration product of the polyester form of the polyglycerol fatty acid ester is preferably, for example, 5% or more, more preferably 10% or more, and still more preferably 20% or more. The area ratio of the peaks corresponding to the polyglycerol and the dehydration product of the polyglycerol is, for example, 1% or more, 5% or more, 10% or more, or 20% or more, and preferably, for example, 80% or less, more preferably 70% or less, and still more preferably 60% or less.

(HPLC Analysis)

The HPLC analysis method for the polyglycerol mono-fatty acid ester product of the present disclosure is not particularly limited, and the measurement can be performed using, for example, the following apparatus and conditions.

- Apparatus: LC-2030C 3D (available from Shimadzu Corporation)
- Column: Two Wakosil 5C18 $\Phi$4.6 mm $\times$ 250 mm (W) columns are connected in series.
- Detector: RID-20A (available from Shimadzu Corporation)
- Oven temperature: 40°C
- Sample: 10% methanol solution
- Sample injection volume: 10 $\mu$l
- Mobile phase flow rate: 0.75 ml/min

Note that the "peaks corresponding to the monoester form, dehydration product of the monoester form, polyester form, and dehydration product of the polyester form of the polyglycerol fatty acid ester, as well as the peaks corresponding to the polyglycerol and the dehydration product of the polyglycerol", refer to, for example, peaks each having its peak top within a retention time of from 6.0 to 20.0 minutes in an HPLC spectrum in the HPLC analysis method described above. Therefore, the area ratio of the peaks is the sum of the peak area ratios within a retention time of from 6.0 to 20.0 minutes in the HPLC spectrum.

The "peaks corresponding to the monoester form, dehydration product of the monoester form, polyester form, and dehydration product of the polyester form of the polyglycerol fatty acid ester" refers to, for example, peaks each having its peak top within a retention time of from 8.2 to 20.0 minutes in the HPLC spectrum in the HPLC analysis method described above. Therefore, the area ratio of the peaks is the sum of the peak area ratios within a retention time of from 8.2 to 20.0 minutes in the HPLC spectrum.

The "peaks corresponding to the polyglycerol and the dehydration product of the polyglycerol" refers to, for example, peaks each having its peak top within a retention time of from 6.0 to 8.2 minutes in the HPLC spectrum in the HPLC analysis method described above. Therefore, the area ratio of the peaks is the sum of the peak area ratios within a retention time of from 6.0 to 8.2 minutes in the HPLC spectrum.

[Method for Producing Polyglycerol Mono-fatty Acid Ester Product]

The polyglycerol mono-fatty acid ester product of the present disclosure may be obtained by any one of (1) a reaction in which glycidol is subjected to addition polymerization with a fatty acid, (2) an esterification reaction of polyglycerol with a fatty acid, or (3) a transesterification reaction of polyglycerol with a fatty acid ester. Among these, the polyglycerol mono-fatty acid ester product obtained by the reaction (1) is preferable in that the proportion of the monoester form becomes high. That is, the polyglycerol mono-fatty acid ester product of the present disclosure is preferably obtained by a production method including carrying out an addition polymerization reaction of glycidol with a fatty acid.

The reaction (1) may be carried out in the presence or absence of a catalyst, and is preferably carried out in the presence of a catalyst (particularly, an acidic catalyst) from the viewpoint of improving the proportion of the monoester form. In particular, when the average degree of polymerization of glycerol of the polyglycerol mono-fatty acid ester product to be obtained is more than 5, the reaction (1) is preferably carried out in the presence of a catalyst. This is because there is a strong tendency for the proportion of the monoester form in the resulting polyglycerol mono-fatty acid ester product to increase. On the contrary, when the average degree of polymerization of glycerol of the polyglycerol mono-fatty acid ester product to be obtained is 5 or less, the reaction (1) is preferably carried out in the presence of a catalyst, but even though the reaction is carried out in the absence of a catalyst, no large difference is observed in the proportion of the monoester form.

The acidic catalyst is not particularly limited, and an organic acid generally used in cosmetics is preferably used as the catalyst, and examples thereof include ascorbic acid; carboxylic acids such as acetic acid, formic acid, citric acid, succinic acid, and adipic acid; and phosphoric acid-based acidic catalysts. The acidic catalyst is preferably an organic acid having a large acid dissociation constant. Among these, a phosphoric acid-based acidic catalyst is preferable from the viewpoint of the purity of the polyglycerol mono-fatty acid ester product.

Examples of the phosphoric acid-based acidic catalyst include phosphoric acids or esters of phosphoric acid, and examples thereof include phosphoric acids such as phosphoric acid, phosphoric anhydride, polyphosphoric acid, orthophosphoric acid, metaphosphoric acid, pyrophosphoric acid, triphosphoric acid, and tetraphosphoric acid; and acidic phosphate esters such as methyl acid phosphate, ethyl acid phosphate, isopropyl acid phosphate, butyl acid phosphate, and 2-ethylhexyl acid phosphate. Among these, phosphoric acid is preferable.

The catalysts may be used singly or in combination of two or more kinds thereof. The blending amount of the catalyst is from 0.001 to 10 parts by weight, and preferably from 0.01 to 5 parts by weight, based on 100 parts by weight of the fatty acid. It is preferable that the blending amount of the catalyst is within the above range, from the viewpoint of increasing the proportion of the monoester form.

When the polyglycerol mono-fatty acid ester product is produced by the reaction (1), the reaction may be carried out, for example, by, while continuously or intermittently supplying glycidol to the fatty acid, continuously or intermittently supplying the acidic catalyst thereto (1-1), or by continuously or intermittently supplying glycidol to the mixture of the fatty acid and the acidic catalyst (1-2). In addition, as an aspect of carrying out the reaction in the absence of a catalyst, the reaction may be carried out by continuously or intermittently supplying glycidol to the fatty acid (1-3).

Specifically describing the above (1-1), it means that a fatty acid is put into a reaction vessel, and while glycidol is continuously or intermittently supplied to the reaction vessel, an acidic catalyst is also continuously or intermittently supplied thereto, whereby an addition polymerization reaction is carried out. That is, it means that glycidol and an acidic catalyst are continuously or intermittently supplied to the fatty acid simultaneously for at least a certain period of time. Examples of the supply method include dropwise addition. Specifically describing the above (1-2), it means that a fatty acid and an acidic catalyst are put into a reaction vessel, mixed as necessary, and glycidol is continuously or intermittently supplied to the reaction vessel, whereby an addition polymerization reaction is carried out. Examples of the supply method include dropwise addition. Specifically describing the above (1-3), it means that a fatty acid is put into a reaction vessel, and glycidol is continuously or intermittently supplied to the reaction vessel, whereby an addition polymerization reaction is carried out. That is, it means that glycidol is continuously or intermittently supplied to the fatty acid simultaneously for at least a certain period of time.

In the reaction (1), not only the formation of the polyglycerol mono-fatty acid ester but also the formation of the polyglycerol may proceed in the reaction system (e.g., in the reaction vessel), but the glycidol concentration in the system can be suppressed to a low level by adding dropwise glycidol continuously or intermittently, and thus the formation of the polyglycerol mono-fatty acid ester tends to become predominant over the formation of the polyglycerol.

The addition reaction with glycidol may be carried out after the acidic catalyst is mixed with the fatty acid, or the reaction may be carried out while the acidic catalyst, together with glycidol as necessary, is continuously or intermittently supplied to the fatty acid, but from the viewpoint of increasing the purity of the resulting polyglycerol mono-fatty acid ester product, it is preferable to carry out the reaction while the acidic catalyst and glycidol are continuously or intermittently supplied to the fatty acid. That is, it is preferable to carry out the reaction (1) by the operation described in the above (1-1). It is considered that this is because the acidic catalyst promotes or facilitates not only the formation of the polyglycerol mono-fatty acid ester but also the formation of the polyglycerol, whereas the formation of the polyglycerol mono-fatty acid ester tends to become particularly predominant over the formation of the polyglycerol when the acidic catalyst is continuously or intermittently supplied, together with glycidol, to the fatty acid. From the same consideration, when the acidic catalyst is supplied, together with glycidol, to the fatty acid at once, the formation of the polyglycerol tends to become predominant over the formation of the polyglycerol mono-fatty acid ester. Therefore, it can be said that it is preferable also from this viewpoint to carry out the reaction by the operation described in the above (1-1).

In the reaction (1), the reaction product may be aged as necessary after the operations described in the above (1-1) to (1-3). The reaction temperature in the reaction (1) is not particularly limited, and is preferably from 50 to 180°C, more preferably from 70 to 160°C, still more preferably from 100 to 140°C, and particularly preferably from 110 to 135°C.

When the reaction temperature is within the above range, a side reaction, such as decomposition of glycidol, tends to be less likely to occur. The reaction is desirably carried out in a nitrogen gas atmosphere, and may be pressurized as necessary.

The reactions (2) and (3) can be carried out, for example, in the presence or absence of a catalyst (alkali catalyst or acid catalyst) under normal pressure or reduced pressure. The charge amount of the polyglycerol and the fatty acid or the polyglycerol and the fatty acid ester is not particularly limited, and can be appropriately selected and used. The reaction temperature is not particularly limited, and is, for example, preferably from 20 to 180°C, more preferably from 30 to 160°C, and still more preferably from 40 to 140°C.

The obtained polyglycerol mono-fatty acid ester may be purified as necessary. The purification method can be carried out using a known method, and is not particularly limited. Examples of the purification method include an adsorption treatment with activated carbon, activated clay, or the like; a treatment under reduced pressure using water vapor, nitrogen, or the like as a carrier gas; washing with an acid or an alkali; and molecular distillation. In addition, impurities can be removed by using liquid-liquid distribution, an adsorbent, a resin, a molecular sieve, a loose reverse osmosis membrane, an ultrafiltration membrane, or the like.

[Cosmetic Composition]

The cosmetic composition of the present disclosure is characterized by containing the polyglycerol mono-fatty acid ester product. In the cosmetic composition, other components can be blended as long as the effects of the invention of the present disclosure are not impaired. Examples of the other components include polyhydric alcohols, sugars, polysaccharides, amino acids, various surfactants (excluding the above-described polyglycerol mono-fatty acid ester product), organic salts, inorganic salts, pH adjusters, chelating agents, antioxidants, bactericides, blood flow promoters, anti-inflammatory agents, ultraviolet absorbers, ultraviolet scattering agents, vitamins, pigments, and perfumes.

The content of the polyglycerol mono-fatty acid ester product in the cosmetic composition is not particularly limited, and is, for example, preferably from 0.01 to 80 wt.%, more preferably from 0.1 to 50 wt.%, still more preferably from 0.1 to 30 wt.%, and particularly preferably from 0.5 to 20 wt.%. When the content of the polyglycerol mono-fatty acid ester product is within the above range, blendability in makeup and spreadability when blended in makeup tend to be sufficient.

Particularly preferred aspects of the cosmetic composition include the following. A cosmetic composition containing:

a polyglycerol mono-fatty acid ester product (A) in which
a fatty acid has from 15 to 25 (preferably from 16 to 18) carbon atoms,
a peak intensity ratio represented by Formula (X) below is 0.31 or more (preferably 0.33 or more, more preferably 0.35 or more, and still more preferably 0.4 or more),
a peak intensity ratio represented by Formula (Y) below is 0.46 or less (preferably 0.37 or less, and more preferably 0.35 or less), and
an average degree of polymerization of glycerol is from 2 to 4 (preferably 4); and
a polyglycerol mono-fatty acid ester product (B) in which
a fatty acid has from 15 to 25 (preferably from 16 to 18) carbon atoms,
a peak intensity ratio represented by Formula (X) below is 0.23 or more (preferably 0.24 or more, more preferably 0.25 or more, and still more preferably 0.26 or more),
a peak intensity ratio represented by Formula (Y) below is 0.46 or less (preferably 0.44 or less), and
an average degree of polymerization of glycerol is from 5 to 12 (preferably from 6 to 10, and more preferably 6 or 10).

$$\text{Formula (X)} = P2/P1$$

$$\text{Formula (Y)} = P3/P1$$

P1: a sum of peak intensities of a monoester form, a dehydration product of the monoester form, a diester form, a dehydration product of the diester form, a triester form, and a dehydration product of the triester form of a polyglycerol fatty acid ester, as well as peak intensities of polyglycerol and a dehydration product of the polyglycerol, when mass spectrometry of the polyglycerol mono-fatty acid ester product is performed using a time-of-flight mass spectrometer
P2: the peak intensity of the monoester form of the polyglycerol fatty acid ester when the mass spectrometry of the polyglycerol mono-fatty acid ester product is performed using the time-of-flight mass spectrometer
P3: a sum of the peak intensities of the polyglycerol and the dehydration product of the polyglycerol when the mass spectrometry of the polyglycerol mono-fatty acid ester product is performed using the time-of-flight mass spectrometer

When the cosmetic composition of the present disclosure takes the above aspect, blendability in makeup, makeup removability, and refreshing feeling on the skin after washing off makeup tend to be good. This tendency becomes more remarkable by combining the polyglycerol mono-fatty acid ester product (A) having a relatively low HLB value with the polyglycerol mono-fatty acid ester product (B) having a relatively high HLB value to adjust the HLB value to a value in the range of from 9 to 13 (particularly from 11 to 12.5). The HLB value in this case is a weighted average of the HLB value of the polyglycerol mono-fatty acid ester product (A) and the HLB value of the polyglycerol mono-fatty acid ester product (B). The HLB value of the polyglycerol mono-fatty acid ester product (A) is not particularly limited, and is, for example, preferably from 9 to 11, more preferably from 9.5 to 10.8, and still more preferably from 10 to 10.6. The HLB value of the polyglycerol mono-fatty acid ester product (B) is not particularly limited, and is, for example, preferably from 11 to 17, more preferably from 11.2 to 15.5, and still more preferably from 11.5 to 15. Therefore, the blending amounts of the polyglycerol mono-fatty acid ester product (A) and the polyglycerol mono-fatty acid ester product (B) in the cosmetic composition of the present disclosure are preferably adjusted so that the HLB value after the combination is from 9 to 13 (particularly from 11 to 12.5) in consideration of the respective HLB values. As the specific blending amounts, the weight ratio of the polyglycerol mono-fatty acid ester product (A) to the polyglycerol mono-fatty acid ester product (B) is, for example, preferably from 20 to 80 : from 80 to 20, more preferably from 30 to 70 : from 70 to 30, and still more preferably from 40 to 60 : from 60 to 40.

Note that the hydrophile-lypophile balance (HLB) value is a value indicating the affinity of a surfactant, such as a polyglycerol mono-fatty acid ester, with water and oil, and can be determined from the following formula by determining an organic value (OV) and an inorganic value (IV) from an organic conceptual diagram.

$$\text{HLB} = \text{IV/OV} \times 10 = \text{IOB (Inorganic Organic Balance)} \times 10$$

The specific product or form of the cosmetic composition of the present disclosure is not particularly limited, and examples thereof include cosmetic liquids, emulsions, skin creams, sunscreen creams, cleansing milks, cleansing lotions, cleansing oils, shampoos, rinses, treatments, hair setting lotions, hair creams, hair oils, hair liquids, hair bleaches, permanent wave liquids, lipsticks, lip glosses, eye glosses, nail glosses, mascaras, and eye shadows.

As applications of the polyglycerol mono-fatty acid ester product of the present disclosure, it can be used not only in the above cosmetic compositions but also in food additives, detergent compositions, and general industrial applications.

Each aspect disclosed in the present specification can be combined with another feature disclosed in the present specification. The configurations, combinations thereof, or the like in each of the embodiments are exemplary, and additions, omissions, replacements, and other changes of the configurations may be made as appropriate without departing from the spirit of the present disclosure. In addition, each of the inventions according to the present disclosure is not limited by the embodiments or the following examples but is limited only by the claims.

Example

Embodiments of the present disclosure will be described in more detail below based on Examples.

(Mass Spectrometry Using Time-of-Flight Mass Spectrometer (TOF-MS))

In Examples and Comparative Examples, polyglycerol mono-fatty acid ester products were subjected to mass spectrometry using the following apparatus and conditions.

- Apparatus: Xevo G2-XSQTof (Waters Corporation)
- Injection method: Injection by infusion (5 $\mu$L/min)
- Measurement mode: Neg
- Cone gas: 50 L/h
- Desolvation gas: 1000 L/h
- Source temp: 120°C
- Desolvation temp: 500°C
- Capillary: 2.0 kV
- Sampling cone: 40
- Source offset:80
- Sample concentration: 25 ppm (methanol)
- MS range: from 50 to 3000

(Measurement of HLB Value)

In Examples and Comparative Examples, the hydrophile-lypophile balance (HLB) values of polyglycerol mono-fatty acid ester products can be determined from the following formula by determining an organic value (OV) and an inorganic value (IV) from the organic conceptual diagram.

$$\text{HLB} = \text{IV/OV} \times 10 = \text{IOB (Inorganic Organic Balance)} \times 10$$

(HPLC Analysis)

In Examples and Comparative Examples, polyglycerol mono-fatty acid ester products were subjected to HPLC analysis using the following apparatus and conditions.

- Apparatus: LC-2030C 3D (available from Shimadzu Corporation)
- Column: Two Wakosil 5C18 $\Phi$4.6 mm $\times$ 250 mm (W) columns were connected in series.
- Detector: RID-20A (available from Shimadzu Corporation)
- Oven temperature: 40°C
- Sample: 10% methanol solution
- Sample injection volume: 10 $\mu$l
- Mobile phase flow rate: 0.75 ml/min

Example 1

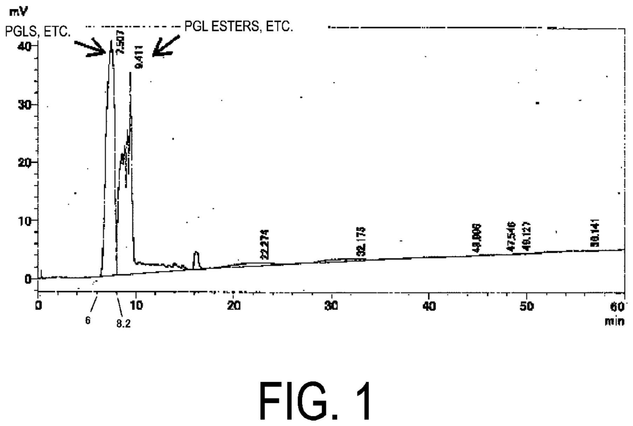

In a 1-liter four-neck flask equipped with a nitrogen-introducing tube, a stirrer, a condenser, a temperature controller, and a dropping cylinder, 0.81 mol (139.7 g) of capric acid was charged and heated to 120°C. Then, 4.86 mol (360.4 g) of glycidol was added dropwise over 10 hours while the reaction temperature was maintained at 120°C, and 0.004 mol (0.4 g) of 42.5% phosphoric acid was added dropwise during the dropwise addition of glycidol. Then, the mixture was aged for 3 hours while being maintained at 120°C. Then, the temperature was raised to 130°C, and the reaction was continued until the oxirane concentration in the system became less than 0.1%. After cooling, the reaction product was taken out to obtain about 500 g of polyglycerol monocaprate having an average degree of polymerization of glycerol of 6.
The obtained polyglycerol monocaprate was subjected to HPLC analysis, and it was found that the area ratio of the peaks corresponding to a monoester form, a dehydration product of the monoester form, a polyester form, and a dehydration product of the polyester form, as well as the peaks corresponding to polyglycerol and a dehydration product of the polyglycerol, each of the peaks existing within a retention time of from 6.0 to 20.0 minutes in the HPLC spectrum, was 93.0%. The area ratio of the peaks corresponding to the monoester form, the dehydration product of the monoester form, the polyester form, and the dehydration product of the polyester form, each of the peaks existing within a retention time of from 8.2 to 20 minutes in the HPLC spectrum, was 47.9%. The area ratio of the peaks corresponding to the polyglycerol and the dehydration product of the polyglycerol, each of the peaks existing within a retention time of from 6.0 to 8.2 minutes in the HPLC spectrum, was 45.1%. The spectrum of HPLC analysis is shown in FIG. 1. In the figure, the peaks corresponding to the monoester form, the dehydration product of the monoester form, the polyester form, and the dehydration product of the polyester form are described as "PGL ESTERS, ETC.", and the peaks corresponding to the polyglycerol and the dehydration product of the polyglycerol as "PGLS, ETC.".
The obtained polyglycerol monocaprate was subjected to mass spectrometry using the time-of-flight mass spectrometer (TOF-MS). Furthermore, the peak intensity ratio (P2/P 1) was calculated according to Formula (X) below, which was found to be 0.325. The peak intensity ratio (P3/P1) was calculated according to Formula (Y) below, which was found to be 0.287.

$$\text{Formula (X)} = \text{P2/P1}$$

$$\text{Formula (Y)} = \text{P3/P1}$$

P1: a sum of peak intensities of a monoester form, a dehydration product of the monoester form, a diester form, a dehydration product of the diester form, a triester form, and a dehydration product of the triester form of a polyglycerol fatty acid ester, as well as peak intensities of polyglycerol and a dehydration product of the polyglycerol, when mass spectrometry of the polyglycerol mono-fatty acid ester product is performed using a time-of-flight mass spectrometer
P2: the peak intensity of the monoester form of the polyglycerol fatty acid ester when the mass spectrometry of the polyglycerol mono-fatty acid ester product is performed using the time-of-flight mass spectrometer

P3: a sum of the peak intensities of the polyglycerol and the dehydration product of the polyglycerol when the mass spectrometry of the polyglycerol mono-fatty acid ester product is performed using the time-of-flight mass spectrometer

The HLB value of the obtained polyglycerol monocaprate was 15.4.

[Method for Calculating Peak Intensity Ratio]

Hereinafter, the method for calculating the peak intensity ratio will be described in detail by dividing into (a) to (e).

(a) The molecular weight and the peak intensity derived from each component contained in the polyglycerol mono-fatty acid ester product were obtained by measurement using the time-of-flight mass spectrometer. The numerical value of each molecular weight was rounded down to an integer by discarding the fractional portion, and the numerical values of the corresponding peak intensities were added up to obtain the peak intensity of the rounded molecular weight. For example, when obtained are such measurement values that the peak intensity is 31 at a molecular weight of "50.0012", 5 at a molecular weight of "50.0041", and 15 at a molecular weight of "50.0241", the sum of the peak intensities (31 + 5 + 15 + ...) within a molecular weight of 50 or more and less than 51 becomes the peak intensity of the above rounded molecular weight "50". The molecular weight was in the range of 50 to 2999. The range of the molecular weight is determined in consideration of the number of carbon atoms of the fatty acid and the degree of polymerization of glycerol of the polyglycerol mono-fatty acid ester product of the present disclosure. That is, it is determined in consideration that there are almost no polyglycerol fatty acid esters that can be contained in the above product and that fall outside the above range of the molecular weight.

Hereinafter, (a) in Example 1 will be described.

By the measurement using the time-of-flight mass spectrometer, the molecular weight and the peak intensity thereof derived from each component contained in the polyglycerol monocaprate obtained in Example 1 were obtained. The numerical values of the respective molecular weights are rounded down to integers by discarding the fractional portions, and the numerical values of the corresponding peak intensities are added up, which are shown in Tables from 1 to 6.

[Table 1]

Table 1

| Mw | Intensity | Mw | Intensity | Mw | Intensity | Mw | Intensity | Mw | Intensity | Mw | Intensity | Mw | Intensity | Mw | Intensity | Mw | Intensity | Mw | Intensity | Mw | Intensity | Mw | Intensity | Mw | Intensity |
|---|---|---|---|---|---|---|---|---|---|---|---|---|---|---|---|---|---|---|---|---|---|---|---|---|---|
| 50 | 1470 | 90 | 590 | 130 | 4500 | 170 | 1660 | 210 | 1990 | 250 | 6600 | 290 | 11100 | 330 | 3260 | 370 | 9730 | 410 | 3120 | 450 | 5750 | 490 | 7790 | 530 | 6960 |
| 51 | 999 | 91 | 2470 | 131 | 4820 | 171 | 390000 | 211 | 2630 | 251 | 4020 | 291 | 6560 | 331 | 7610 | 371 | 5350 | 411 | 9630 | 451 | 12000 | 491 | 22800 | 531 | 5900 |
| 52 | 997 | 92 | 998 | 132 | 1500 | 172 | 53700 | 212 | 2070 | 252 | 3000 | 292 | 4240 | 332 | 6230 | 372 | 3840 | 412 | 4010 | 452 | 4470 | 492 | 17600 | 532 | 4690 |
| 53 | 832 | 93 | 1910 | 133 | 8500 | 173 | 5970 | 213 | 4550 | 253 | 4200 | 293 | 11500 | 333 | 10100 | 373 | 5110 | 413 | 7520 | 453 | 65900 | 493 | 41200 | 533 | 13400 |
| 54 | 1080 | 94 | 38700 | 134 | 2550 | 174 | 3770 | 214 | 4740 | 254 | 3890 | 294 | 3600 | 334 | 4140 | 374 | 3540 | 414 | 4890 | 454 | 17500 | 494 | 13200 | 534 | 7290 |
| 55 | 1220 | 95 | 5450 | 135 | 1650 | 175 | 1530 | 215 | 4580 | 255 | 150000 | 295 | 33900 | 335 | 6330 | 375 | 4110 | 415 | 15900 | 455 | 9620 | 495 | 10800 | 535 | 24100 |
| 56 | 920 | 96 | 54000 | 136 | 1180 | 176 | 2290 | 216 | 3590 | 256 | 27600 | 296 | 6050 | 336 | 3080 | 376 | 3990 | 416 | 6100 | 456 | 6160 | 496 | 6460 | 536 | 9410 |
| 57 | 1150 | 97 | 2070 | 137 | 1690 | 177 | 2960 | 217 | 3550 | 257 | 7170 | 297 | 12000 | 337 | 8550 | 377 | 7930 | 417 | 7260 | 457 | 5340 | 497 | 14000 | 537 | 7360 |
| 58 | 1530 | 98 | 3850 | 138 | 5430 | 178 | 2650 | 218 | 2340 | 258 | 4300 | 298 | 4300 | 338 | 3840 | 378 | 3930 | 418 | 16600 | 458 | 4450 | 498 | 14100 | 538 | 4750 |
| 59 | 4610 | 99 | 1890 | 139 | 4090 | 179 | 1460 | 219 | 3660 | 259 | 3510 | 299 | 6710 | 339 | 24300 | 379 | 79400 | 419 | 58500 | 459 | 7720 | 499 | 38100 | 539 | 10900 |
| 60 | 990 | 100 | 961 | 140 | 1440 | 180 | 1880 | 220 | 7060 | 260 | 2420 | 300 | 2790 | 340 | 8590 | 380 | 19000 | 420 | 14100 | 460 | 4440 | 500 | 13600 | 540 | 5430 |
| 61 | 1580 | 101 | 5440 | 141 | 2330 | 181 | 2310 | 221 | 13200 | 261 | 2980 | 301 | 6730 | 341 | 13100 | 381 | 6780 | 421 | 10300 | 461 | 26700 | 501 | 16100 | 541 | 190000 |
| 62 | 1540 | 102 | 822 | 142 | 947 | 182 | 2710 | 222 | 3430 | 262 | 2090 | 302 | 3220 | 342 | 4120 | 382 | 4550 | 422 | 7830 | 462 | 8460 | 502 | 7720 | 542 | 54900 |
| 63 | 662 | 103 | 2010 | 143 | 14400 | 183 | 5260 | 223 | 3040 | 263 | 6470 | 303 | 5370 | 343 | 5770 | 383 | 10300 | 423 | 19400 | 463 | 9020 | 503 | 33800 | 543 | 21000 |
| 64 | 1050 | 104 | 938 | 144 | 2130 | 184 | 2140 | 224 | 1680 | 264 | 7980 | 304 | 3020 | 344 | 14500 | 384 | 5440 | 424 | 7390 | 464 | 4520 | 504 | 10500 | 544 | 7270 |
| 65 | 798 | 105 | 999 | 145 | 3040 | 185 | 6310 | 225 | 4600 | 265 | 8520 | 305 | 16000 | 345 | 52200 | 385 | 7940 | 425 | 5750 | 465 | 10400 | 505 | 14100 | 545 | 6150 |
| 66 | 1000 | 106 | 692 | 146 | 806 | 186 | 6150 | 226 | 2770 | 266 | 6860 | 306 | 3930 | 346 | 11600 | 386 | 3670 | 426 | 4390 | 466 | 4610 | 506 | 8050 | 546 | 5310 |
| 67 | 1100 | 107 | 722 | 147 | 3810 | 187 | 2600 | 227 | 11400 | 267 | 3990 | 307 | 4720 | 347 | 5940 | 387 | 26000 | 427 | 6250 | 467 | 217000 | 507 | 6140 | 547 | 18400 |
| 68 | 826 | 108 | 1610 | 148 | 6940 | 188 | 1490 | 228 | 3550 | 268 | 2760 | 308 | 3350 | 348 | 6110 | 388 | 7360 | 428 | 4240 | 468 | 54300 | 508 | 4980 | 548 | 11300 |
| 69 | 1160 | 109 | 2830 | 149 | 10000 | 189 | 2000 | 229 | 3940 | 269 | 6020 | 309 | 4600 | 349 | 18700 | 389 | 8230 | 429 | 47100 | 469 | 20400 | 509 | 26400 | 549 | 25600 |
| 70 | 808 | 110 | 1020 | 150 | 1330 | 190 | 1370 | 230 | 3830 | 270 | 5920 | 310 | 4810 | 350 | 5610 | 390 | 3820 | 430 | 12900 | 470 | 5870 | 510 | 9620 | 550 | 8350 |
| 71 | 1140 | 111 | 2830 | 151 | 1390 | 191 | 3790 | 231 | 2330 | 271 | 11500 | 311 | 24400 | 351 | 5670 | 391 | 8140 | 431 | 14500 | 471 | 4670 | 511 | 8840 | 551 | 8980 |
| 72 | 1110 | 112 | 1620 | 152 | 861 | 192 | 2990 | 232 | 5950 | 272 | 3130 | 312 | 8960 | 352 | 3790 | 392 | 4930 | 432 | 6500 | 472 | 3550 | 512 | 3730 | 552 | 5550 |
| 73 | 4340 | 113 | 1600 | 153 | 1320 | 193 | 2200 | 233 | 8510 | 273 | 2510 | 313 | 27800 | 353 | 5120 | 393 | 187000 | 433 | 5580 | 473 | 9240 | 513 | 36700 | 553 | 7180 |
| 74 | 1110 | 114 | 1270 | 154 | 10800 | 194 | 2590 | 234 | 9710 | 274 | 2530 | 314 | 7240 | 354 | 3030 | 394 | 43000 | 434 | 3650 | 474 | 8240 | 514 | 14400 | 554 | 4890 |
| 75 | 2320 | 115 | 11500 | 155 | 2720 | 195 | 4280 | 235 | 3200 | 275 | 3370 | 315 | 4110 | 355 | 40100 | 395 | 20900 | 435 | 20200 | 475 | 17300 | 515 | 11300 | 555 | 7340 |
| 76 | 10300 | 116 | 3450 | 156 | 4580 | 196 | 2020 | 236 | 3870 | 276 | 2950 | 316 | 1810 | 356 | 7900 | 396 | 5350 | 436 | 7590 | 476 | 7410 | 516 | 7480 | 556 | 4780 |
| 77 | 1830 | 117 | 7530 | 157 | 11200 | 197 | 6930 | 237 | 3280 | 277 | 5380 | 317 | 4930 | 357 | 12900 | 397 | 5380 | 437 | 7900 | 477 | 9100 | 517 | 52400 | 557 | 8850 |
| 78 | 1790 | 118 | 1740 | 158 | 3200 | 198 | 22200 | 238 | 1870 | 278 | 2590 | 318 | 2570 | 358 | 5260 | 398 | 3920 | 438 | 3650 | 478 | 3650 | 518 | 15700 | 558 | 6040 |
| 79 | 3020 | 119 | 797 | 159 | 4310 | 199 | 14700 | 239 | 19900 | 279 | 3210 | 319 | 37600 | 359 | 5500 | 399 | 4590 | 439 | 50500 | 479 | 12000 | 519 | 13000 | 559 | 9240 |
| 80 | 1210 | 120 | 2460 | 160 | 6170 | 200 | 4930 | 240 | 3730 | 280 | 4030 | 320 | 11400 | 360 | 3450 | 400 | 4050 | 440 | 18800 | 480 | 5310 | 520 | 5080 | 560 | 5670 |
| 81 | 962 | 121 | 2160 | 161 | 2830 | 201 | 3760 | 241 | 5630 | 281 | 16700 | 321 | 13600 | 361 | 15700 | 401 | 7180 | 441 | 9890 | 481 | 8030 | 521 | 7230 | 561 | 7760 |
| 82 | 934 | 122 | 1030 | 162 | 5910 | 202 | 1910 | 242 | 3870 | 282 | 4620 | 322 | 4220 | 362 | 7270 | 402 | 5400 | 442 | 4610 | 482 | 3220 | 522 | 4340 | 562 | 5190 |
| 83 | 987 | 123 | 762 | 163 | 18500 | 203 | 2630 | 243 | 3700 | 283 | 69500 | 323 | 4500 | 363 | 10300 | 403 | 6150 | 443 | 43600 | 483 | 7550 | 523 | 10800 | 563 | 14200 |
| 84 | 1180 | 124 | 862 | 164 | 38800 | 204 | 2940 | 244 | 1640 | 284 | 16500 | 324 | 5110 | 364 | 4790 | 404 | 3710 | 444 | 11600 | 484 | 4430 | 524 | 7000 | 564 | 7090 |
| 85 | 1140 | 125 | 1540 | 165 | 11000 | 205 | 2820 | 245 | 4730 | 285 | 6700 | 325 | 29300 | 365 | 35300 | 405 | 7820 | 445 | 6790 | 485 | 8720 | 525 | 9660 | 565 | 12400 |
| 86 | 739 | 126 | 22600 | 166 | 3770 | 206 | 1090 | 246 | 8290 | 286 | 2990 | 326 | 7870 | 366 | 11100 | 406 | 5000 | 446 | 4610 | 486 | 4600 | 526 | 5110 | 566 | 11500 |
| 87 | 2550 | 127 | 4760 | 167 | 1360 | 207 | 4640 | 247 | 17300 | 287 | 2940 | 327 | 4920 | 367 | 15100 | 407 | 9610 | 447 | 5510 | 487 | 6930 | 527 | 44100 | 567 | 26200 |
| 88 | 804 | 128 | 1430 | 168 | 1250 | 208 | 2250 | 248 | 7660 | 288 | 10300 | 328 | 3210 | 368 | 5140 | 408 | 4310 | 448 | 3220 | 488 | 4030 | 528 | 13600 | 568 | 12100 |
| 89 | 2850 | 129 | 5270 | 169 | 2520 | 209 | 1690 | 249 | 3080 | 289 | 64000 | 329 | 3740 | 369 | 48800 | 409 | 5840 | 449 | 8020 | 489 | 13300 | 529 | 11700 | 569 | 9210 |

[Table 2]

Table 2

| Mw | Intensity | Mw | Intensity | Mw | Intensity | Mw | Intensity | Mw | Intensity | Mw | Intensity | Mw | Intensity | Mw | Intensity | Mw | Intensity | Mw | Intensity | Mw | Intensity | Mw | Intensity | Mw | Intensity |
|---|---|---|---|---|---|---|---|---|---|---|---|---|---|---|---|---|---|---|---|---|---|---|---|---|---|
| 570 | 6410 | 610 | 9670 | 650 | 12000 | 690 | 46100 | 730 | 7310 | 770 | 13700 | 810 | 10300 | 850 | 7060 | 890 | 8010 | 930 | 7930 | 970 | 7910 | 1010 | 9670 | 1050 | 6610 |
| 571 | 10700 | 611 | 7870 | 651 | 14900 | 691 | 19400 | 731 | 15500 | 771 | 19900 | 811 | 10700 | 851 | 9440 | 891 | 8700 | 931 | 8480 | 971 | 9240 | 1011 | 12200 | 1051 | 8840 |
| 572 | 10400 | 612 | 5400 | 652 | 8830 | 692 | 8010 | 732 | 8570 | 772 | 11500 | 812 | 8660 | 852 | 8330 | 892 | 8510 | 932 | 7690 | 972 | 9320 | 1012 | 8850 | 1052 | 8050 |
| 573 | 32500 | 613 | 10400 | 653 | 11400 | 693 | 7700 | 733 | 7820 | 773 | 7170 | 813 | 23900 | 853 | 8840 | 893 | 15800 | 933 | 14300 | 973 | 11100 | 1013 | 8380 | 1053 | 12000 |
| 574 | 11900 | 614 | 6440 | 654 | 5480 | 694 | 5560 | 734 | 7030 | 774 | 7560 | 814 | 13200 | 854 | 7620 | 894 | 10300 | 934 | 9150 | 974 | 9810 | 1014 | 8390 | 1054 | 10500 |
| 575 | 34400 | 615 | 156000 | 655 | 7350 | 695 | 16700 | 735 | 17400 | 775 | 7840 | 815 | 10400 | 855 | 8890 | 895 | 10000 | 935 | 13300 | 975 | 9410 | 1015 | 10500 | 1055 | 10500 |
| 576 | 15400 | 616 | 49800 | 656 | 5240 | 696 | 14200 | 736 | 10600 | 776 | 6740 | 816 | 7610 | 856 | 6990 | 896 | 9000 | 936 | 13500 | 976 | 8530 | 1016 | 9370 | 1056 | 7720 |
| 577 | 26000 | 617 | 21900 | 657 | 17100 | 697 | 22400 | 737 | 11800 | 777 | 8510 | 817 | 9030 | 857 | 7890 | 897 | 10800 | 937 | 12400 | 977 | 13200 | 1017 | 12200 | 1057 | 9570 |
| 578 | 10400 | 618 | 7640 | 658 | 9850 | 698 | 11300 | 738 | 8730 | 778 | 6780 | 818 | 6550 | 858 | 7560 | 898 | 8400 | 938 | 8440 | 978 | 8870 | 1018 | 9930 | 1058 | 9160 |
| 579 | 11600 | 619 | 7030 | 659 | 8890 | 699 | 7990 | 739 | 27800 | 779 | 11100 | 819 | 15900 | 859 | 14700 | 899 | 12500 | 939 | 9910 | 979 | 10700 | 1019 | 9890 | 1059 | 32200 |
| 580 | 6450 | 620 | 5290 | 660 | 7160 | 700 | 5420 | 740 | 15400 | 780 | 7840 | 820 | 9680 | 860 | 9570 | 900 | 7640 | 940 | 9730 | 980 | 9150 | 1020 | 7630 | 1060 | 21000 |
| 581 | 8400 | 621 | 17700 | 661 | 20000 | 701 | 7790 | 741 | 11000 | 781 | 9490 | 821 | 10900 | 861 | 15900 | 901 | 8890 | 941 | 9330 | 981 | 17700 | 1021 | 10200 | 1061 | 12100 |
| 582 | 5260 | 622 | 11500 | 662 | 9560 | 702 | 6580 | 742 | 7470 | 782 | 7850 | 822 | 8310 | 862 | 13400 | 902 | 7710 | 942 | 10800 | 982 | 10900 | 1022 | 8620 | 1062 | 7970 |
| 583 | 22700 | 623 | 23100 | 663 | 9330 | 703 | 8140 | 743 | 8010 | 783 | 9990 | 823 | 13400 | 863 | 14900 | 903 | 10500 | 943 | 14400 | 983 | 8880 | 1023 | 10600 | 1063 | 9800 |
| 584 | 10200 | 624 | 10100 | 664 | 7940 | 704 | 5540 | 744 | 6700 | 784 | 7220 | 824 | 8830 | 864 | 8810 | 904 | 8440 | 944 | 10500 | 984 | 8000 | 1024 | 8900 | 1064 | 8060 |
| 585 | 10100 | 625 | 8910 | 665 | 34300 | 705 | 9840 | 745 | 14200 | 785 | 14100 | 825 | 13900 | 865 | 9850 | 905 | 17100 | 945 | 9430 | 985 | 9570 | 1025 | 8580 | 1065 | 13600 |
| 586 | 5660 | 626 | 4950 | 666 | 13800 | 706 | 7830 | 746 | 7830 | 786 | 9930 | 826 | 8100 | 866 | 9200 | 906 | 10800 | 946 | 6800 | 986 | 8580 | 1026 | 7820 | 1066 | 10700 |
| 587 | 25100 | 627 | 5610 | 667 | 5610 | 707 | 8320 | 747 | 8420 | 787 | 16900 | 827 | 7910 | 867 | 11400 | 907 | 10500 | 947 | 9300 | 987 | 14400 | 1027 | 10400 | 1067 | 12300 |
| 588 | 10800 | 628 | 4830 | 668 | 6430 | 708 | 7220 | 748 | 7380 | 788 | 15200 | 828 | 6940 | 868 | 10500 | 908 | 8820 | 948 | 8200 | 988 | 10700 | 1028 | 8200 | 1068 | 10900 |
| 589 | 10600 | 629 | 7230 | 669 | 9570 | 709 | 9540 | 749 | 15300 | 789 | 14200 | 829 | 11200 | 869 | 15500 | 909 | 9680 | 949 | 9570 | 989 | 11200 | 1029 | 9570 | 1069 | 10500 |
| 590 | 8440 | 630 | 5010 | 670 | 5600 | 710 | 5880 | 750 | 8700 | 790 | 8890 | 830 | 6660 | 870 | 10900 | 910 | 8760 | 950 |  | 990 | 8760 | 1030 | 9100 | 1070 | 8540 |
| 591 | 44300 | 631 | 8640 | 671 | 10900 | 711 | 15500 | 751 | 13400 | 791 | 8240 | 831 | 16200 | 871 | 11700 | 911 | 54500 | 951 |  | 991 | 8410 | 1031 | 12100 | 1071 | 9220 |
| 592 | 12200 | 632 | 6220 | 672 | 7500 | 712 | 9160 | 752 | 8620 | 792 | 10500 | 832 | 10600 | 872 | 8300 | 912 | 28800 | 952 |  | 992 | 8520 | 1032 | 10600 | 1072 | 6290 |
| 593 | 11900 | 633 | 8110 | 673 | 9710 | 713 | 16100 | 753 | 7820 | 793 | 9360 | 833 | 9360 | 873 | 11100 | 913 | 17100 | 953 |  | 993 | 11200 | 1033 | 10700 | 1073 | 8120 |
| 594 | 5920 | 634 | 5770 | 674 | 7050 | 714 | 14700 | 754 | 6280 | 794 | 9900 | 834 | 7020 | 874 | 8190 | 914 | 9570 | 954 |  | 994 | 10800 | 1034 | 9510 | 1074 | 7020 |
| 595 | 8030 | 635 | 7260 | 675 | 19600 | 715 | 16400 | 755 | 10500 | 795 | 16400 | 835 | 11900 | 875 | 10700 | 915 | 8970 | 955 |  | 995 | 8050 | 1035 | 14500 | 1075 | 10300 |
| 596 | 4690 | 636 | 5150 | 676 | 10200 | 716 | 7370 | 756 | 7730 | 796 | 11700 | 836 | 7370 | 876 | 7340 | 916 | 8130 | 956 |  | 996 | 7740 | 1036 | 11800 | 1076 | 9680 |
| 597 | 9230 | 637 | 15200 | 677 | 16400 | 717 | 9490 | 757 | 18900 | 797 | 11100 | 837 | 74900 | 877 | 10000 | 917 | 13700 | 957 |  | 997 | 9120 | 1037 | 10200 | 1077 | 7990 |
| 598 | 6840 | 638 | 8140 | 678 | 8530 | 718 | 6710 | 758 | 10700 | 798 | 7450 | 838 | 3600 | 878 | 8830 | 918 | 10600 | 958 |  | 998 | 8160 | 1038 | 9520 | 1078 | 7970 |
| 599 | 9550 | 639 | 20900 | 679 | 7040 | 719 | 10500 | 759 | 9370 | 799 | 10200 | 839 | 17000 | 879 | 10900 | 919 | 16900 | 959 |  | 999 | 13000 | 1039 | 9120 | 1079 | 10900 |
| 600 | 6360 | 640 | 15100 | 680 | 5210 | 720 | 9720 | 760 | 6390 | 800 | 7970 | 840 | 9240 | 880 | 9220 | 920 | 10800 | 960 |  | 1000 | 10600 | 1040 | 8630 | 1080 | 7160 |
| 601 | 27000 | 641 | 16900 | 681 | 9900 | 721 | 20700 | 761 | 11700 | 801 | 11200 | 841 | 8760 | 881 | 9060 | 921 | 8690 | 961 |  | 1001 | 9990 | 1041 | 11200 | 1081 | 12000 |
| 602 | 10300 | 642 | 8460 | 682 | 6830 | 722 | 8700 | 762 | 8400 | 802 | 7430 | 842 | 6630 | 882 | 7890 | 922 | 6200 | 962 |  | 1002 | 7890 | 1042 | 8660 | 1082 | 9700 |
| 603 | 18500 | 643 | 8470 | 683 | 21000 | 723 | 13500 | 763 | 94000 | 803 | 9280 | 843 | 14400 | 883 | 14700 | 923 | 8910 | 963 |  | 1003 | 11600 | 1043 | 10300 | 1083 | 12700 |
| 604 | 11200 | 644 | 6720 | 684 | 9010 | 724 | 8230 | 764 | 40000 | 804 | 6970 | 844 | 12900 | 884 | 10900 | 924 | 6910 | 964 |  | 1004 | 7860 | 1044 | 9160 | 1084 | 11100 |
| 605 | 6740 | 645 | 10000 | 685 | 9410 | 725 | 12100 | 765 | 18600 | 805 | 12400 | 845 | 18000 | 885 | 9960 | 925 | 9140 | 965 |  | 1005 | 43600 | 1045 | 9720 | 1085 | 12600 |
| 606 | 4470 | 646 | 10500 | 686 | 6440 | 726 | 9110 | 766 | 9210 | 806 | 9730 | 846 | 11700 | 886 | 9210 | 926 | 8330 | 966 |  | 1006 | 25600 | 1046 | 8450 | 1086 | 9720 |
| 607 | 12300 | 647 | 24500 | 687 | 11200 | 727 | 10500 | 767 | 7980 | 807 | 10400 | 847 | 7930 | 887 | 21000 | 927 | 9700 | 967 |  | 1007 | 15400 | 1047 | 10600 | 1087 | 10200 |
| 608 | 5990 | 648 | 11400 | 688 | 8870 | 728 | 7160 | 768 | 6360 | 808 | 7810 | 848 | 6460 | 888 | 121000 | 928 | 8120 | 968 |  | 1008 | 9820 | 1048 | 9120 | 1088 | 9250 |
| 609 | 23600 | 649 | 21100 | 689 | 121000 | 729 | 9560 | 769 | 17500 | 809 | 15500 | 849 | 8330 | 889 | 10800 | 929 | 8440 | 969 |  | 1009 | 9460 | 1049 | 9440 | 1089 | 10000 |

[Table 3]

Table 3

| Mw | Intensity | Mw | Intensity | Mw | Intensity | Mw | Intensity | Mw | Intensity | Mw | Intensity | Mw | Intensity | Mw | Intensity | Mw | Intensity | Mw | Intensity | Mw | Intensity | Mw | Intensity | Mw | Intensity |
|---|---|---|---|---|---|---|---|---|---|---|---|---|---|---|---|---|---|---|---|---|---|---|---|---|---|
| 1090 | 8270 | 1130 | 6900 | 1170 | 7160 | 1210 | 9360 | 1250 | 9990 | 1290 | 10200 | 1330 | 8050 | 1370 | 7390 | 1410 | 7180 | 1450 | 8260 | 1490 | 7250 | 1530 | 6890 | 1570 | 6610 |
| 1091 | 11400 | 1131 | 9430 | 1171 | 9550 | 1211 | 9290 | 1251 | 8840 | 1291 | 9870 | 1331 | 7900 | 1371 | 7550 | 1411 | 8670 | 1451 | 7830 | 1491 | 7950 | 1531 | 8980 | 1571 | 6830 |
| 1092 | 10200 | 1132 | 8720 | 1172 | 9070 | 1212 | 8180 | 1252 | 6660 | 1292 | 8630 | 1332 | 8560 | 1372 | 8450 | 1412 | 7790 | 1452 | 7520 | 1492 | 7740 | 1532 | 7190 | 1572 | 7050 |
| 1093 | 9250 | 1133 | 26500 | 1173 | 8990 | 1213 | 9330 | 1253 | 10600 | 1293 | 7810 | 1333 | 9150 | 1373 | 7250 | 1413 | 8790 | 1453 | 8870 | 1493 | 8030 | 1533 | 7320 | 1573 | 7180 |
| 1094 | 8310 | 1134 | 17500 | 1174 | 8170 | 1214 | 8790 | 1254 | 8690 | 1294 | 8410 | 1334 | 7890 | 1374 | 8340 | 1414 | 8830 | 1454 | 8040 | 1494 | 7660 | 1534 | 6120 | 1574 | 6130 |
| 1095 | 8300 | 1135 | 13200 | 1175 | 8630 | 1215 | 11600 | 1255 | 9130 | 1295 | 8950 | 1335 | 8420 | 1375 | 8.90 | 1415 | 8510 | 1455 | 8550 | 1495 | 7570 | 1535 | 7980 | 1575 | 8330 |
| 1096 | 8180 | 1136 | 9090 | 1176 | 9330 | 1216 | 9410 | 1256 | 6900 | 1296 | 7090 | 1336 | 7590 | 1376 | 5930 | 1416 | 8360 | 1456 | 8080 | 1496 | 8680 | 1536 | 7360 | 1576 | 7080 |
| 1097 | 9270 | 1137 | 10300 | 1177 | 9750 | 1217 | 9910 | 1257 | 10200 | 1297 | 7450 | 1337 | 9630 | 1377 | 8560 | 1417 | 8610 | 1457 | 8070 | 1497 | 6950 | 1537 | 7150 | 1577 | 7860 |
| 1098 | 8680 | 1138 | 8870 | 1178 | 8170 | 1218 | 8600 | 1258 | 8740 | 1298 | 8400 | 1338 | 8180 | 1378 | 7960 | 1418 | 8120 | 1458 | 8740 | 1498 | 7320 | 1538 | 7260 | 1578 | 8110 |
| 1099 | 8600 | 1139 | 10200 | 1179 | 9950 | 1219 | 8720 | 1259 | 9040 | 1299 | 9120 | 1339 | 9120 | 1379 | 7420 | 1419 | 8600 | 1459 | 6350 | 1499 | 7640 | 1539 | 7160 | 1579 | 8600 |
| 1100 | 7020 | 1140 | 10500 | 1180 | 7840 | 1220 | 8550 | 1260 | 9070 | 1300 | 9050 | 1340 | 9050 | 1380 | 8210 | 1420 | 7710 | 1460 | 8770 | 1500 | 7380 | 1540 | 8740 | 1580 | 6540 |
| 1101 | 8890 | 1141 | 12100 | 1181 | 9880 | 1221 | 8700 | 1261 | 10000 | 1301 | 8970 | 1341 | 8030 | 1381 | 9040 | 1421 | 7760 | 1461 | 8710 | 1501 | 8420 | 1541 | 7390 | 1581 | 7450 |
| 1102 | 8240 | 1142 | 8530 | 1182 | 8170 | 1222 | 8010 | 1262 | 9130 | 1302 | 6880 | 1342 | 7260 | 1382 | 7560 | 1422 | 7670 | 1462 | 7720 | 1502 | 6940 | 1542 | 7440 | 1582 | 7910 |
| 1103 | 9590 | 1143 | 9760 | 1183 | 11200 | 1223 | 8360 | 1263 | 9380 | 1303 | 10000 | 1343 | 8680 | 1383 | 7600 | 1423 | 8460 | 1463 | 8300 | 1503 | 9090 | 1543 | 7500 | 1583 | 8600 |
| 1104 | 8660 | 1144 | 7970 | 1184 | 10400 | 1224 | 8900 | 1264 | 7210 | 1304 | 8390 | 1344 | 8170 | 1384 | 7460 | 1424 | 8630 | 1464 | 6900 | 1504 | 7920 | 1544 | 6010 | 1584 | 7320 |
| 1105 | 10300 | 1145 | 9150 | 1185 | 9990 | 1225 | 9170 | 1265 | 10200 | 1305 | 9510 | 1345 | 8660 | 1385 | 9170 | 1425 | 8270 | 1465 | 8720 | 1505 | 8190 | 1545 | 8690 | 1585 | 8920 |
| 1106 | 9080 | 1146 | 6900 | 1186 | 8140 | 1226 | 7810 | 1266 | 8850 | 1306 | 8800 | 1346 | 6990 | 1386 | 8130 | 1426 | 8330 | 1466 | 6370 | 1506 | 6990 | 1546 | 6250 | 1586 | 8830 |
| 1107 | 10300 | 1147 | 8960 | 1187 | 8310 | 1227 | 7870 | 1267 | 9030 | 1307 | 8750 | 1347 | 7250 | 1387 | 8740 | 1427 | 7970 | 1467 | 7950 | 1507 | 7070 | 1547 | 7250 | 1587 | 7910 |
| 1108 | 7860 | 1148 | 6900 | 1188 | 8360 | 1228 | 6360 | 1268 | 8320 | 1308 | 9680 | 1348 | 7700 | 1388 | 8860 | 1428 | 8520 | 1468 | 6930 | 1508 | 7440 | 1548 | 8000 | 1588 | 7470 |
| 1109 | 14000 | 1149 | 8810 | 1189 | 10800 | 1229 | 11000 | 1269 | 7740 | 1309 | 7100 | 1349 | 7870 | 1389 | 8320 | 1429 | 11300 | 1469 | 7540 | 1509 | 7120 | 1549 | 7100 | 1589 | 6570 |
| 1110 | 8930 | 1150 | 8020 | 1190 | 9410 | 1230 | 8170 | 1270 | 7490 | 1310 | 7500 | 1350 | 9860 | 1390 | 6220 | 1430 | 9940 | 1470 | 6760 | 1510 | 8690 | 1550 | 6510 | 1590 | 7460 |
| 1111 | 10300 | 1151 | 9350 | 1191 | 7770 | 1231 | 9740 | 1271 | 8080 | 1311 | 9700 | 1351 | 7580 | 1391 | 7860 | 1431 | 10200 | 1471 | 7670 | 1511 | 6110 | 1551 | 6740 | 1591 | 7480 |
| 1112 | 7800 | 1152 | 8040 | 1192 | 7320 | 1232 | 9130 | 1272 | 6810 | 1312 | 9020 | 1352 | 6900 | 1392 | 8520 | 1432 | 8280 | 1472 | 8330 | 1512 | 7540 | 1552 | 7370 | 1592 | 8080 |
| 1113 | 9480 | 1153 | 9360 | 1193 | 8940 | 1233 | 8930 | 1273 | 9480 | 1313 | 9430 | 1353 | 8060 | 1393 | 7500 | 1433 | 8120 | 1473 | 7700 | 1513 | 7300 | 1553 | 8010 | 1593 | 8370 |
| 1114 | 8210 | 1154 | 9010 | 1194 | 8410 | 1234 | 8420 | 1274 | 7910 | 1314 | 8030 | 1354 | 9430 | 1394 | 7980 | 1434 | 7840 | 1474 | 7900 | 1514 | 6990 | 1554 | 6950 | 1594 | 7260 |
| 1115 | 11400 | 1155 | 10400 | 1195 | 9340 | 1235 | 10400 | 1275 | 10900 | 1315 | 8650 | 1355 | 13200 | 1395 | 8120 | 1435 | 9970 | 1475 | 7760 | 1515 | 8130 | 1555 | 7350 | 1595 | 7130 |
| 1116 | 9250 | 1156 | 9950 | 1196 | 7990 | 1236 | 8120 | 1276 | 8660 | 1316 | 7590 | 1356 | 10700 | 1396 | 7840 | 1436 | 8440 | 1476 | 8050 | 1516 | 7940 | 1556 | 7690 | 1596 | 6720 |
| 1117 | 9610 | 1157 | 9970 | 1197 | 7980 | 1237 | 9760 | 1277 | 9800 | 1317 | 8710 | 1357 | 9350 | 1397 | 8540 | 1437 | 9710 | 1477 | 8460 | 1517 | 7950 | 1557 | 7750 | 1597 | 6990 |
| 1118 | 7340 | 1158 | 8430 | 1198 | 6790 | 1238 | 10300 | 1278 | 8300 | 1318 | 8670 | 1358 | 8380 | 1398 | 8210 | 1438 | 8440 | 1478 | 6730 | 1518 | 7980 | 1558 | 6930 | 1598 | 6990 |
| 1119 | 10200 | 1159 | 11500 | 1199 | 8890 | 1239 | 10300 | 1279 | 8540 | 1319 | 9070 | 1359 | 8340 | 1399 | 7390 | 1439 | 8240 | 1479 | 8160 | 1519 | 7490 | 1559 | 7810 | 1599 | 7230 |
| 1120 | 9240 | 1160 | 8980 | 1200 | 9330 | 1240 | 8420 | 1280 | 9170 | 1320 | 8520 | 1360 | 7880 | 1400 | 7720 | 1440 | 8200 | 1480 | 8930 | 1520 | 8140 | 1560 | 8660 | 1600 | 7110 |
| 1121 | 10000 | 1161 | 7690 | 1201 | 13000 | 1241 | 9140 | 1281 | 16600 | 1321 | 10100 | 1361 | 9460 | 1401 | 9190 | 1441 | 7180 | 1481 | 7560 | 1521 | 7150 | 1561 | 7070 | 1601 | 7620 |
| 1122 | 8910 | 1162 | 8230 | 1202 | 8990 | 1242 | 7920 | 1282 | 12600 | 1322 | 8110 | 1362 | 8540 | 1402 | 7380 | 1442 | 8320 | 1482 | 6570 | 1522 | 7670 | 1562 | 7920 | 1602 | 7420 |
| 1123 | 8350 | 1163 | 10600 | 1203 | 8950 | 1243 | 8990 | 1283 | 10700 | 1323 | 7950 | 1363 | 9770 | 1403 | 8890 | 1443 | 7510 | 1483 | 7060 | 1523 | 7470 | 1563 | 6990 | 1603 | 6430 |
| 1124 | 7150 | 1164 | 9460 | 1204 | 7020 | 1244 | 7140 | 1284 | 9350 | 1324 | 8860 | 1364 | 7960 | 1404 | 7990 | 1444 | 8930 | 1484 | 8260 | 1524 | 7130 | 1564 | 6530 | 1604 | 6720 |
| 1125 | 10500 | 1165 | 10700 | 1205 | 9580 | 1245 | 10400 | 1285 | 9830 | 1325 | 7620 | 1365 | 9060 | 1405 | 7610 | 1445 | 8130 | 1485 | 7690 | 1525 | 8440 | 1565 | 8020 | 1605 | 7570 |
| 1126 | 8550 | 1166 | 9300 | 1206 | 8650 | 1246 | 8890 | 1286 | 9060 | 1326 | 9600 | 1366 | 8530 | 1406 | 8390 | 1446 | 6800 | 1486 | 8620 | 1526 | 7410 | 1566 | 7030 | 1606 | 7870 |
| 1127 | 10600 | 1167 | 8080 | 1207 | 17800 | 1247 | 8660 | 1287 | 8920 | 1327 | 9290 | 1367 | 7990 | 1407 | 7990 | 1447 | 7670 | 1487 | 8850 | 1527 | 8140 | 1567 | 7640 | 1607 | 6790 |
| 1128 | 8960 | 1168 | 8070 | 1208 | 15100 | 1248 | 7080 | 1288 | 9070 | 1328 | 8330 | 1368 | 8130 | 1408 | 8360 | 1448 | 7900 | 1488 | 8300 | 1528 | 7690 | 1568 | 7030 | 1608 | 7460 |
| 1129 | 9200 | 1169 | 9290 | 1209 | 12000 | 1249 | 8580 | 1289 | 10800 | 1329 | 9120 | 1369 | 7900 | 1409 | 8560 | 1449 | 8290 | 1489 | 8110 | 1529 | 9160 | 1569 | 6080 | 1609 | 6240 |

18

[Table 4]

Table 4

| Mw | Intensity | Mw | Intensity | Mw | Intensity | Mw | Intensity | Mw | Intensity | Mw | Intensity | Mw | Intensity | Mw | Intensity | Mw | Intensity | Mw | Intensity | Mw | Intensity | Mw | Intensity | Mw | Intensity |
|---|---|---|---|---|---|---|---|---|---|---|---|---|---|---|---|---|---|---|---|---|---|---|---|---|---|
| 1610 | 7590 | 1650 | 7080 | 1690 | 7650 | 1730 | 6460 | 1770 | 6200 | 1810 | 6750 | 1850 | 5020 | 1890 | 6430 | 1930 | 5710 | 1970 | 5320 | 2010 | 5800 | 2050 | 3930 | 2090 | 5850 |
| 1611 | 7290 | 1651 | 8800 | 1691 | 6740 | 1731 | 6480 | 1771 | 6850 | 1811 | 6010 | 1851 | 6090 | 1891 | 6540 | 1931 | 5450 | 1971 | 5680 | 2011 | 4740 | 2051 | 4390 | 2091 | 5120 |
| 1612 | 6240 | 1652 | 7800 | 1692 | 6320 | 1732 | 6490 | 1772 | 5530 | 1812 | 5920 | 1852 | 6580 | 1892 | 5710 | 1932 | 6230 | 1972 | 6230 | 2012 | 4210 | 2052 | 5730 | 2092 | 5110 |
| 1613 | 7040 | 1653 | 7810 | 1693 | 7300 | 1733 | 6340 | 1773 | 6050 | 1813 | 6660 | 1853 | 6140 | 1893 | 6130 | 1933 | 5490 | 1973 | 4930 | 2013 | 5730 | 2053 | 6120 | 2093 | 5260 |
| 1614 | 8160 | 1654 | 7330 | 1694 | 6280 | 1734 | 7390 | 1774 | 6780 | 1814 | 6450 | 1854 | 5400 | 1894 | 7080 | 1934 | 5550 | 1974 | 4860 | 2014 | 5560 | 2054 | 5010 | 2094 | 4700 |
| 1615 | 8210 | 1655 | 7640 | 1695 | 6520 | 1735 | 6950 | 1775 | 6300 | 1815 | 6730 | 1855 | 7260 | 1895 | 6020 | 1935 | 5930 | 1975 | 5870 | 2015 | 6020 | 2055 | 6090 | 2095 | 5150 |
| 1616 | 7870 | 1656 | 6610 | 1696 | 6540 | 1736 | 8610 | 1776 | 6940 | 1816 | 7120 | 1856 | 5750 | 1896 | 6680 | 1936 | 6760 | 1976 | 5310 | 2016 | 5710 | 2056 | 5220 | 2096 | 5740 |
| 1617 | 7050 | 1657 | 6970 | 1697 | 6590 | 1737 | 5420 | 1777 | 6100 | 1817 | 7310 | 1857 | 6450 | 1897 | 6360 | 1937 | 5600 | 1977 | 6100 | 2017 | 6330 | 2057 | 5650 | 2097 | 5940 |
| 1618 | 7260 | 1658 | 8490 | 1698 | 7630 | 1738 | 5820 | 1778 | 6420 | 1818 | 6270 | 1858 | 5730 | 1898 | 6290 | 1938 | 5480 | 1978 | 5560 | 2018 | 5090 | 2058 | 5030 | 2098 | 4470 |
| 1619 | 6900 | 1659 | 7470 | 1699 | 7370 | 1739 | 6600 | 1779 | 6600 | 1819 | 5290 | 1859 | 6710 | 1899 | 6480 | 1939 | 6440 | 1979 | 5930 | 2019 | 6730 | 2059 | 6090 | 2099 | 4990 |
| 1620 | 6970 | 1660 | 6880 | 1700 | 5880 | 1740 | 6810 | 1780 | 6770 | 1820 | 7030 | 1860 | 7290 | 1900 | 5840 | 1940 | 5290 | 1980 | 5300 | 2020 | 7010 | 2060 | 5680 | 2100 | 5500 |
| 1621 | 7470 | 1661 | 7170 | 1701 | 7810 | 1741 | 6970 | 1781 | 6490 | 1821 | 5720 | 1861 | 5130 | 1901 | 6600 | 1941 | 5920 | 1981 | 6000 | 2021 | 5940 | 2061 | 6020 | 2101 | 6510 |
| 1622 | 7230 | 1662 | 6080 | 1702 | 7370 | 1742 | 6380 | 1782 | 6660 | 1822 | 5060 | 1862 | 6330 | 1902 | 6420 | 1942 | 5880 | 1982 | 5290 | 2022 | 5810 | 2062 | 5470 | 2102 | 4890 |
| 1623 | 6480 | 1663 | 7310 | 1703 | 6820 | 1743 | 6630 | 1783 | 6280 | 1823 | 5580 | 1863 | 6240 | 1903 | 6230 | 1943 | 6320 | 1983 | 6500 | 2023 | 5900 | 2063 | 5450 | 2103 | 5390 |
| 1624 | 6760 | 1664 | 8120 | 1704 | 7200 | 1744 | 6940 | 1784 | 6480 | 1824 | 6710 | 1864 | 5410 | 1904 | 6510 | 1944 | 5460 | 1984 | 5670 | 2024 | 5760 | 2064 | 6090 | 2104 | 5160 |
| 1625 | 7910 | 1665 | 7150 | 1705 | 7700 | 1745 | 6830 | 1785 | 7460 | 1825 | 7030 | 1865 | 4830 | 1905 | 6020 | 1945 | 5540 | 1985 | 6150 | 2025 | 4860 | 2065 | 5400 | 2105 | 4660 |
| 1626 | 7200 | 1666 | 7520 | 1706 | 7450 | 1746 | 7080 | 1786 | 6260 | 1826 | 7000 | 1866 | 5090 | 1906 | 5930 | 1946 | 5530 | 1986 | 5690 | 2026 | 5230 | 2066 | 5360 | 2106 | 5440 |
| 1627 | 7430 | 1667 | 5590 | 1707 | 8590 | 1747 | 6260 | 1787 | 5610 | 1827 | 5950 | 1867 | 6200 | 1907 | 5300 | 1947 | 5110 | 1987 | 5340 | 2027 | 5980 | 2067 | 5740 | 2107 | 4840 |
| 1628 | 7250 | 1668 | 7110 | 1708 | 6310 | 1748 | 6730 | 1788 | 7170 | 1828 | 5600 | 1868 | 5690 | 1908 | 5710 | 1948 | 5870 | 1988 | 5940 | 2028 | 6430 | 2068 | 4560 | 2108 | 6040 |
| 1629 | 7780 | 1669 | 7940 | 1709 | 7760 | 1749 | 6290 | 1789 | 7470 | 1829 | 6560 | 1869 | 6580 | 1909 | 6360 | 1949 | 6210 | 1989 | 6270 | 2029 | 4940 | 2069 | 4820 | 2109 | 5220 |
| 1630 | 6620 | 1670 | 5830 | 1710 | 7540 | 1750 | 6600 | 1790 | 6190 | 1830 | 7000 | 1870 | 5910 | 1910 | 5770 | 1950 | 6260 | 1990 | 6280 | 2030 | 5290 | 2070 | 4630 | 2110 | 5930 |
| 1631 | 8110 | 1671 | 7260 | 1711 | 7590 | 1751 | 7330 | 1791 | 6680 | 1831 | 6040 | 1871 | 6540 | 1911 | 6090 | 1951 | 6340 | 1991 | 5610 | 2031 | 5210 | 2071 | 6130 | 2111 | 4780 |
| 1632 | 7890 | 1672 | 7420 | 1712 | 7140 | 1752 | 7750 | 1792 | 6960 | 1832 | 5390 | 1872 | 7020 | 1912 | 5460 | 1952 | 6520 | 1992 | 5420 | 2032 | 4650 | 2072 | 5000 | 2112 | 5050 |
| 1633 | 6400 | 1673 | 7390 | 1713 | 6150 | 1753 | 7300 | 1793 | 6220 | 1833 | 6200 | 1873 | 6360 | 1913 | 5430 | 1953 | 6420 | 1993 | 4930 | 2033 | 5300 | 2073 | 6180 | 2113 | 5340 |
| 1634 | 6820 | 1674 | 6020 | 1714 | 6690 | 1754 | 5890 | 1794 | 6840 | 1834 | 6740 | 1874 | 6510 | 1914 | 5400 | 1954 | 6360 | 1994 | 5810 | 2034 | 5910 | 2074 | 4510 | 2114 | 5390 |
| 1635 | 7210 | 1675 | 6560 | 1715 | 7180 | 1755 | 7890 | 1795 | 5550 | 1835 | 6220 | 1875 | 6260 | 1915 | 6950 | 1955 | 7460 | 1995 | 5870 | 2035 | 5540 | 2075 | 5750 | 2115 | 6330 |
| 1636 | 8410 | 1676 | 6210 | 1716 | 8090 | 1756 | 7410 | 1796 | 6340 | 1836 | 5860 | 1876 | 5940 | 1916 | 6130 | 1956 | 5240 | 1996 | 5240 | 2036 | 5910 | 2076 | 5990 | 2116 | 4470 |
| 1637 | 7050 | 1677 | 7220 | 1717 | 8490 | 1757 | 7430 | 1797 | 7110 | 1837 | 6720 | 1877 | 5930 | 1917 | 5290 | 1957 | 6520 | 1997 | 5830 | 2037 | 6360 | 2077 | 5410 | 2117 | 5010 |
| 1638 | 6680 | 1678 | 7210 | 1718 | 7280 | 1758 | 7240 | 1798 | 6840 | 1838 | 6900 | 1878 | 6050 | 1918 | 5500 | 1958 | 4780 | 1998 | 6470 | 2038 | 4800 | 2078 | 5110 | 2118 | 5530 |
| 1639 | 6220 | 1679 | 6360 | 1719 | 6440 | 1759 | 6280 | 1799 | 6600 | 1839 | 5770 | 1879 | 5940 | 1919 | 6200 | 1959 | 5450 | 1999 | 5820 | 2039 | 5520 | 2079 | 5230 | 2119 | 5700 |
| 1640 | 7370 | 1680 | 6990 | 1720 | 7470 | 1760 | 6570 | 1800 | 6370 | 1840 | 5670 | 1880 | 7080 | 1920 | 5590 | 1960 | 5910 | 2000 | 5570 | 2040 | 5210 | 2080 | 5650 | 2120 | 4630 |
| 1641 | 7400 | 1681 | 7500 | 1721 | 7080 | 1761 | 6460 | 1801 | 5990 | 1841 | 5760 | 1881 | 6010 | 1921 | 6060 | 1961 | 6440 | 2001 | 5230 | 2041 | 5160 | 2081 | 4880 | 2121 | 6170 |
| 1642 | 6390 | 1682 | 7340 | 1722 | 7220 | 1762 | 6350 | 1802 | 6210 | 1842 | 6930 | 1882 | 6800 | 1922 | 6520 | 1962 | 5540 | 2002 | 5620 | 2042 | 6120 | 2082 | 5350 | 2122 | 4490 |
| 1643 | 7480 | 1683 | 7860 | 1723 | 7320 | 1763 | 6700 | 1803 | 5760 | 1843 | 5710 | 1883 | 5920 | 1923 | 5570 | 1963 | 5530 | 2003 | 5310 | 2043 | 5000 | 2083 | 4950 | 2123 | 4700 |
| 1644 | 6490 | 1684 | 7070 | 1724 | 8320 | 1764 | 7460 | 1804 | 7170 | 1844 | 5700 | 1884 | 6160 | 1924 | 5690 | 1964 | 6220 | 2004 | 6160 | 2044 | 4960 | 2084 | 4960 | 2124 | 5490 |
| 1645 | 6880 | 1685 | 7430 | 1725 | 6360 | 1765 | 7580 | 1805 | 6010 | 1845 | 6110 | 1885 | 5640 | 1925 | 6460 | 1965 | 6090 | 2005 | 6410 | 2045 | 5420 | 2085 | 4890 | 2125 | 4260 |
| 1646 | 7600 | 1686 | 5740 | 1726 | 6110 | 1766 | 6910 | 1806 | 6320 | 1846 | 5390 | 1886 | 5930 | 1926 | 5660 | 1966 | 5300 | 2006 | 4950 | 2046 | 5910 | 2086 | 6020 | 2126 | 4680 |
| 1647 | 7300 | 1687 | 6120 | 1727 | 6510 | 1767 | 7820 | 1807 | 6330 | 1847 | 7360 | 1887 | 5920 | 1927 | 6650 | 1967 | 7340 | 2007 | 5880 | 2047 | 5400 | 2087 | 5090 | 2127 | 4380 |
| 1648 | 5640 | 1688 | 6270 | 1728 | 6740 | 1768 | 7110 | 1808 | 7600 | 1848 | 5700 | 1888 | 6290 | 1928 | 5060 | 1968 | 6360 | 2008 | 6350 | 2048 | 6270 | 2088 | 4280 | 2128 | 5310 |
| 1649 | 8560 | 1689 | 8240 | 1729 | 6740 | 1769 | 5550 | 1809 | 7150 | 1849 | 6700 | 1889 | 5560 | 1929 | 5000 | 1969 | 5760 | 2009 | 4610 | 2049 | 4880 | 2089 | 5850 | 2129 | 5850 |

[Table 5]

Table 5

| Mw | Intensity | Mw | Intensity | Mw | Intensity | Mw | Intensity | Mw | Intensity | Mw | Intensity | Mw | Intensity | Mw | Intensity | Mw | Intensity | Mw | Intensity | Mw | Intensity | Mw | Intensity | Mw | Intensity |
|---|---|---|---|---|---|---|---|---|---|---|---|---|---|---|---|---|---|---|---|---|---|---|---|---|---|
| 2130 | 4500 | 2170 | 4480 | 2210 | 4120 | 2250 | 4590 | 2290 | 4560 | 2330 | 3890 | 2370 | 3770 | 2410 | 4150 | 2450 | 4160 | 2490 | 3650 | 2530 | 4660 | 2570 | 3410 | 2610 | 2900 |
| 2131 | 4590 | 2171 | 4800 | 2211 | 5180 | 2251 | 5150 | 2291 | 4560 | 2331 | 4180 | 2371 | 4630 | 2411 | 3810 | 2451 | 3610 | 2491 | 3980 | 2531 | 3340 | 2571 | 3970 | 2611 | 3290 |
| 2132 | 4800 | 2172 | 5600 | 2212 | 5430 | 2252 | 4510 | 2292 | 3960 | 2332 | 4270 | 2372 | 5090 | 2412 | 3080 | 2452 | 3630 | 2492 | 3820 | 2532 | 2650 | 2572 | 3200 | 2612 | 2990 |
| 2133 | 5890 | 2173 | 5310 | 2213 | 4290 | 2253 | 4280 | 2293 | 5030 | 2333 | 3930 | 2373 | 4330 | 2413 | 4000 | 2453 | 4140 | 2493 | 4090 | 2533 | 4010 | 2573 | 2800 | 2613 | 3410 |
| 2134 | 5180 | 2174 | 5710 | 2214 | 4710 | 2254 | 4180 | 2294 | 4620 | 2334 | 4250 | 2374 | 4090 | 2414 | 3370 | 2454 | 3840 | 2494 | 3040 | 2534 | 3220 | 2574 | 4060 | 2614 | 3530 |
| 2135 | 4780 | 2175 | 3900 | 2215 | 4910 | 2255 | 5570 | 2295 | 5110 | 2335 | 4030 | 2375 | 3990 | 2415 | 5310 | 2455 | 3070 | 2495 | 3370 | 2535 | 3250 | 2575 | 3380 | 2615 | 3890 |
| 2136 | 6130 | 2176 | 4580 | 2216 | 4810 | 2256 | 4230 | 2296 | 4640 | 2336 | 4010 | 2376 | 3580 | 2416 | 4030 | 2456 | 3800 | 2496 | 3080 | 2536 | 3870 | 2576 | 3700 | 2616 | 4000 |
| 2137 | 5070 | 2177 | 4120 | 2217 | 6560 | 2257 | 4290 | 2297 | 4510 | 2337 | 4070 | 2377 | 4000 | 2417 | 3140 | 2457 | 4080 | 2497 | 4670 | 2537 | 3420 | 2577 | 3980 | 2617 | 3640 |
| 2138 | 5070 | 2178 | 4410 | 2218 | 4290 | 2258 | 4320 | 2298 | 4620 | 2338 | 4280 | 2378 | 4460 | 2418 | 3290 | 2458 | 3750 | 2498 | 4010 | 2538 | 4010 | 2578 | 3390 | 2618 | 2780 |
| 2139 | 5250 | 2179 | 4730 | 2219 | 4400 | 2259 | 5120 | 2299 | 3710 | 2339 | 4180 | 2379 | 5020 | 2419 | 3320 | 2459 | 3910 | 2499 | 3070 | 2539 | 3260 | 2579 | 3770 | 2619 | 3860 |
| 2140 | 4320 | 2180 | 4770 | 2220 | 4450 | 2260 | 4360 | 2300 | 4600 | 2340 | 3870 | 2380 | 3690 | 2420 | 3810 | 2460 | 3430 | 2500 | 2740 | 2540 | 3400 | 2580 | 4130 | 2620 | 3300 |
| 2141 | 5350 | 2181 | 4670 | 2221 | 3900 | 2261 | 4190 | 2301 | 4630 | 2341 | 4710 | 2381 | 3330 | 2421 | 3760 | 2461 | 3950 | 2501 | 3890 | 2541 | 3890 | 2581 | 3410 | 2621 | 3500 |
| 2142 | 4620 | 2182 | 3760 | 2222 | 5350 | 2262 | 4060 | 2302 | 4670 | 2342 | 4880 | 2382 | 4970 | 2422 | 3250 | 2462 | 4960 | 2502 | 3270 | 2542 | 3270 | 2582 | 2900 | 2622 | 2710 |
| 2143 | 5400 | 2183 | 5260 | 2223 | 4360 | 2263 | 4060 | 2303 | 5010 | 2343 | 3330 | 2383 | 4440 | 2423 | 3590 | 2463 | 4320 | 2503 | 3820 | 2543 | 3820 | 2583 | 2370 | 2623 | 3300 |
| 2144 | 4850 | 2184 | 4620 | 2224 | 4560 | 2264 | 4450 | 2304 | 3680 | 2344 | 4520 | 2384 | 3500 | 2424 | 4710 | 2464 | 4150 | 2504 | 3840 | 2544 | 3840 | 2584 | 2790 | 2624 | 3930 |
| 2145 | 5470 | 2185 | 6200 | 2225 | 4920 | 2265 | 3940 | 2305 | 4990 | 2345 | 5300 | 2385 | 3940 | 2425 | 4270 | 2465 | 3410 | 2505 | 3300 | 2545 | 3300 | 2585 | 3730 | 2625 | 3230 |
| 2146 | 5230 | 2186 | 4580 | 2226 | 4730 | 2266 | 3610 | 2306 | 3560 | 2346 | 3510 | 2386 | 5080 | 2426 | 4740 | 2466 | 3190 | 2506 | 3590 | 2546 | 3590 | 2586 | 2580 | 2626 | 3740 |
| 2147 | 4040 | 2187 | 4490 | 2227 | 4570 | 2267 | 5460 | 2307 | 4680 | 2347 | 4060 | 2387 | 4120 | 2427 | 3950 | 2467 | 3860 | 2507 | 3290 | 2547 | 3290 | 2587 | 3800 | 2627 | 3350 |
| 2148 | 5290 | 2188 | 4540 | 2228 | 5200 | 2268 | 4860 | 2308 | 4380 | 2348 | 4170 | 2388 | 4400 | 2428 | 4010 | 2468 | 3780 | 2508 | 3780 | 2548 | 3780 | 2588 | 3040 | 2628 | 2980 |
| 2149 | 4800 | 2189 | 5310 | 2229 | 5050 | 2269 | 4710 | 2309 | 4300 | 2349 | 3590 | 2389 | 3750 | 2429 | 5210 | 2469 | 4310 | 2509 | 3620 | 2549 | 3620 | 2589 | 3440 | 2629 | 3270 |
| 2150 | 4170 | 2190 | 5060 | 2230 | 4670 | 2270 | 4250 | 2310 | 4120 | 2350 | 4570 | 2390 | 3790 | 2430 | 4480 | 2470 | 3240 | 2510 | 4110 | 2550 | 3920 | 2590 | 3720 | 2630 | 2800 |
| 2151 | 4690 | 2191 | 4170 | 2231 | 4490 | 2271 | 4320 | 2311 | 4190 | 2351 | 4350 | 2391 | 4420 | 2431 | 3830 | 2471 | 4410 | 2511 | 3290 | 2551 | 3290 | 2591 | 3620 | 2631 | 3250 |
| 2152 | 4140 | 2192 | 4700 | 2232 | 4660 | 2272 | 4540 | 2312 | 4540 | 2352 | 4690 | 2392 | 3870 | 2432 | 3820 | 2472 | 3820 | 2512 | 3420 | 2552 | 3420 | 2592 | 3350 | 2632 | 2790 |
| 2153 | 4580 | 2193 | 4710 | 2233 | 4710 | 2273 | 4660 | 2313 | 4660 | 2353 | 4540 | 2393 | 3280 | 2433 | 3730 | 2473 | 4140 | 2513 | 3750 | 2553 | 3750 | 2593 | 3000 | 2633 | 2850 |
| 2154 | 4670 | 2194 | 4580 | 2234 | 4000 | 2274 | 4040 | 2314 | 4040 | 2354 | 4050 | 2394 | 4310 | 2434 | 3650 | 2474 | 4120 | 2514 | 3870 | 2554 | 3870 | 2594 | 2430 | 2634 | 2860 |
| 2155 | 4740 | 2195 | 5180 | 2235 | 3940 | 2275 | 4950 | 2315 | 4950 | 2355 | 4430 | 2395 | 4230 | 2435 | 4330 | 2475 | 3130 | 2515 | 4210 | 2555 | 3650 | 2595 | 4060 | 2635 | 3620 |
| 2156 | 3980 | 2196 | 4960 | 2236 | 5120 | 2276 | 5050 | 2316 | 5050 | 2356 | 4250 | 2396 | 3990 | 2436 | 4450 | 2476 | 4100 | 2516 | 3180 | 2556 | 3650 | 2596 | 3050 | 2636 | 2890 |
| 2157 | 5170 | 2197 | 4530 | 2237 | 4090 | 2277 | 3980 | 2317 | 3980 | 2357 | 4650 | 2397 | 3970 | 2437 | 4140 | 2477 | 3400 | 2517 | 4150 | 2557 | 3890 | 2597 | 3420 | 2637 | 2660 |
| 2158 | 5230 | 2198 | 3680 | 2238 | 4170 | 2278 | 3870 | 2318 | 3870 | 2358 | 4190 | 2398 | 3050 | 2438 | 4050 | 2478 | 4090 | 2518 | 3580 | 2558 | 3560 | 2598 | 2850 | 2638 | 4170 |
| 2159 | 4740 | 2199 | 4200 | 2239 | 4490 | 2279 | 4010 | 2319 | 4010 | 2359 | 3530 | 2399 | 3750 | 2439 | 4340 | 2479 | 4680 | 2519 | 3450 | 2559 | 3450 | 2599 | 3880 | 2639 | 3360 |
| 2160 | 4770 | 2200 | 5000 | 2240 | 4660 | 2280 | 4070 | 2320 | 4070 | 2360 | 3800 | 2400 | 4850 | 2440 | 4140 | 2480 | 4270 | 2520 | 3520 | 2560 | 3070 | 2600 | 2840 | 2640 | 3680 |
| 2161 | 4990 | 2201 | 4310 | 2241 | 4480 | 2281 | 5190 | 2321 | 5190 | 2361 | 4590 | 2401 | 4000 | 2441 | 5060 | 2481 | 3810 | 2521 | 3880 | 2561 | 4080 | 2601 | 3090 | 2641 | 2810 |
| 2162 | 4620 | 2202 | 4630 | 2242 | 5260 | 2282 | 3650 | 2322 | 3650 | 2362 | 4160 | 2402 | 3460 | 2442 | 3920 | 2482 | 3450 | 2522 | 3130 | 2562 | 3240 | 2602 | 3960 | 2642 | 3850 |
| 2163 | 5900 | 2203 | 5270 | 2243 | 4180 | 2283 | 4310 | 2323 | 4310 | 2363 | 3800 | 2403 | 4120 | 2443 | 3170 | 2483 | 3570 | 2523 | 3880 | 2563 | 3130 | 2603 | 2820 | 2643 | 3380 |
| 2164 | 4310 | 2204 | 5370 | 2244 | 3830 | 2284 | 3680 | 2324 | 3680 | 2364 | 3940 | 2404 | 4090 | 2444 | 4490 | 2484 | 3970 | 2524 | 3470 | 2564 | 3240 | 2604 | 3860 | 2644 | 3100 |
| 2165 | 5090 | 2205 | 4530 | 2245 | 4170 | 2285 | 4500 | 2325 | 4500 | 2365 | 4700 | 2405 | 4370 | 2445 | 4090 | 2485 | 3470 | 2525 | 3200 | 2565 | 3360 | 2605 | 3370 | 2645 | 2760 |
| 2166 | 3860 | 2206 | 5020 | 2246 | 4670 | 2286 | 4060 | 2326 | 4060 | 2366 | 4080 | 2406 | 4240 | 2446 | 3230 | 2486 | 3900 | 2526 | 3760 | 2566 | 3130 | 2606 | 3670 | 2646 | 3530 |
| 2167 | 4390 | 2207 | 5750 | 2247 | 4290 | 2287 | 3760 | 2327 | 3760 | 2367 | 4460 | 2407 | 4300 | 2447 | 2790 | 2487 | 3250 | 2527 | 3880 | 2567 | 3340 | 2607 | 3020 | 2647 | 2900 |
| 2168 | 4980 | 2208 | 3970 | 2248 | 3630 | 2288 | 4050 | 2328 | 4050 | 2368 | 3570 | 2408 | 4300 | 2448 | 2820 | 2488 | 4710 | 2528 | 3480 | 2568 | 3580 | 2608 | 4140 | 2648 | 3330 |
| 2169 | 4050 | 2209 | 3100 | 2249 | 4940 | 2289 | 4100 | 2329 | 4100 | 2369 | 4420 | 2409 | 3890 | 2449 | 4000 | 2489 | 3980 | 2529 | 3680 | 2569 | 3270 | 2609 | 3930 | 2649 | 3210 |

[Table 6]

Table 6

| Mw | 2650 | 2651 | 2652 | 2653 | 2654 | 2655 | 2656 | 2657 | 2658 | 2659 | 2660 | 2661 | 2662 | 2663 | 2664 | 2665 | 2666 | 2667 | 2668 | 2669 | 2670 | 2671 | 2672 | 2673 | 2674 | 2675 | 2676 | 2677 | 2678 | 2679 | 2680 | 2681 | 2682 | 2683 | 2684 | 2685 | 2686 | 2687 | 2688 | 2689 |
|---|---|---|---|---|---|---|---|---|---|---|---|---|---|---|---|---|---|---|---|---|---|---|---|---|---|---|---|---|---|---|---|---|---|---|---|---|---|---|---|---|
| Intensity | 3530 | 3710 | 3230 | 2860 | 2910 | 2980 | 3540 | 3370 | 2710 | 3350 | 3140 | 2670 | 3260 | 4410 | 2690 | 2880 | 2860 | 2920 | 3520 | 3840 | 2700 | 3130 | 3030 | 2700 | 3140 | 3110 | 3100 | 2730 | 3560 | 3550 | 3050 | 2810 | 3460 | 3870 | 2840 | 3120 | 2760 | 3420 | 3220 | 3240 |

| Mw | 2690 | 2691 | 2692 | 2693 | 2694 | 2695 | 2696 | 2697 | 2698 | 2699 | 2700 | 2701 | 2702 | 2703 | 2704 | 2705 | 2706 | 2707 | 2708 | 2709 | 2710 | 2711 | 2712 | 2713 | 2714 | 2715 | 2716 | 2717 | 2718 | 2719 | 2720 | 2721 | 2722 | 2723 | 2724 | 2725 | 2726 | 2727 | 2728 | 2729 |
|---|---|---|---|---|---|---|---|---|---|---|---|---|---|---|---|---|---|---|---|---|---|---|---|---|---|---|---|---|---|---|---|---|---|---|---|---|---|---|---|---|
| Intensity | 3440 | 2740 | 2680 | 3190 | 3400 | 3580 | 3410 | 3180 | 2760 | 2710 | 3290 | 3070 | 3070 | 3440 | 3260 | 2460 | 2880 | 3580 | 2250 | 3500 | 2830 | 3410 | 2960 | 3410 | 2320 | 2820 | 2120 | 3080 | 3740 | 2910 | 2890 | 2840 | 2080 | 3810 | 3310 | 2800 | 2440 | 3130 | 2700 | 2270 |

| Mw | 2730 | 2731 | 2732 | 2733 | 2734 | 2735 | 2736 | 2737 | 2738 | 2739 | 2740 | 2741 | 2742 | 2743 | 2744 | 2745 | 2746 | 2747 | 2748 | 2749 | 2750 | 2751 | 2752 | 2753 | 2754 | 2755 | 2756 | 2757 | 2758 | 2759 | 2760 | 2761 | 2762 | 2763 | 2764 | 2765 | 2766 | 2767 | 2768 | 2769 |
|---|---|---|---|---|---|---|---|---|---|---|---|---|---|---|---|---|---|---|---|---|---|---|---|---|---|---|---|---|---|---|---|---|---|---|---|---|---|---|---|---|
| Intensity | 2630 | 3110 | 2660 | 2710 | 3130 | 3020 | 2770 | 2850 | 3060 | 2910 | 2880 | 2930 | 2580 | 3580 | 3060 | 2510 | 3130 | 3110 | 3170 | 2870 | 3210 | 3210 | 2080 | 2550 | 2330 | 3470 | 2860 | 2770 | 2900 | 2280 | 2400 | 2600 | 2650 | 3060 | 3900 | 2970 | 2790 | 2480 | 2680 | 2520 |

| Mw | 2770 | 2771 | 2772 | 2773 | 2774 | 2775 | 2776 | 2777 | 2778 | 2779 | 2780 | 2781 | 2782 | 2783 | 2784 | 2785 | 2786 | 2787 | 2788 | 2789 | 2790 | 2791 | 2792 | 2793 | 2794 | 2795 | 2796 | 2797 | 2798 | 2799 | 2800 | 2801 | 2802 | 2803 | 2804 | 2805 | 2806 | 2807 | 2808 | 2809 |
|---|---|---|---|---|---|---|---|---|---|---|---|---|---|---|---|---|---|---|---|---|---|---|---|---|---|---|---|---|---|---|---|---|---|---|---|---|---|---|---|---|
| Intensity | 3210 | 2740 | 2220 | 2710 | 3260 | 2700 | 2720 | 2990 | 2730 | 2080 | 2670 | 2720 | 2280 | 2610 | 2470 | 2130 | 2800 | 2070 | 2330 | 2420 | 3280 | 2410 | 3360 | 2260 | 2820 | 2830 | 3170 | 2420 | 3090 | 2400 | 2890 | 2980 | 2800 | 3220 | 2170 | 3280 | 2300 | 2130 | 2260 | 2790 |

| Mw | 2810 | 2811 | 2812 | 2813 | 2814 | 2815 | 2816 | 2817 | 2818 | 2819 | 2820 | 2821 | 2822 | 2823 | 2824 | 2825 | 2826 | 2827 | 2828 | 2829 | 2830 | 2831 | 2832 | 2833 | 2834 | 2835 | 2836 | 2837 | 2838 | 2839 | 2840 | 2841 | 2842 | 2843 | 2844 | 2845 | 2846 | 2847 | 2848 | 2849 |
|---|---|---|---|---|---|---|---|---|---|---|---|---|---|---|---|---|---|---|---|---|---|---|---|---|---|---|---|---|---|---|---|---|---|---|---|---|---|---|---|---|
| Intensity | 2370 | 2360 | 3190 | 2940 | 2850 | 2920 | 3060 | 3050 | 3200 | 2430 | 2780 | 3060 | 2600 | 2190 | 2560 | 2200 | 2760 | 2790 | 2900 | 2690 | 2930 | 2790 | 2860 | 2990 | 2770 | 2760 | 2320 | 2290 | 2980 | 2710 | 3250 | 3480 | 2870 | 2720 | 2840 | 2400 | 2290 | 2250 | 2710 | 3330 |

| Mw | 2850 | 2851 | 2852 | 2853 | 2854 | 2855 | 2856 | 2857 | 2858 | 2859 | 2860 | 2861 | 2862 | 2863 | 2864 | 2865 | 2866 | 2867 | 2868 | 2869 | 2870 | 2871 | 2872 | 2873 | 2874 | 2875 | 2876 | 2877 | 2878 | 2879 | 2880 | 2881 | 2882 | 2883 | 2884 | 2885 | 2886 | 2887 | 2888 | 2889 |
|---|---|---|---|---|---|---|---|---|---|---|---|---|---|---|---|---|---|---|---|---|---|---|---|---|---|---|---|---|---|---|---|---|---|---|---|---|---|---|---|---|
| Intensity | 2640 | 2340 | 3830 | 2440 | 2600 | 2210 | 2420 | 2580 | 1470 | 2500 | 2520 | 2330 | 1970 | 2760 | 2680 | 2920 | 3490 | 2630 | 3370 | 2470 | 2630 | 2410 | 2830 | 2340 | 2190 | 2290 | 2410 | 3220 | 2140 | 2690 | 2540 | 2180 | 2670 | 2760 | 2410 | 3040 | 2930 | 2370 | 3630 | 2880 |

| Mw | 2890 | 2891 | 2892 | 2893 | 2894 | 2895 | 2896 | 2897 | 2898 | 2899 | 2900 | 2901 | 2902 | 2903 | 2904 | 2905 | 2906 | 2907 | 2908 | 2909 | 2910 | 2911 | 2912 | 2913 | 2914 | 2915 | 2916 | 2917 | 2918 | 2919 | 2920 | 2921 | 2922 | 2923 | 2924 | 2925 | 2926 | 2927 | 2928 | 2929 |
|---|---|---|---|---|---|---|---|---|---|---|---|---|---|---|---|---|---|---|---|---|---|---|---|---|---|---|---|---|---|---|---|---|---|---|---|---|---|---|---|---|
| Intensity | 2810 | 2230 | 2710 | 2530 | 3020 | 2320 | 1500 | 2400 | 2710 | 2070 | 2260 | 2730 | 2740 | 2320 | 2420 | 3160 | 2830 | 2450 | 2850 | 2200 | 2640 | 2840 | 1900 | 2700 | 3170 | 3020 | 2910 | 2660 | 2420 | 2320 | 2170 | 2280 | 2460 | 3040 | 2110 | 2180 | 1840 | 2440 | 2680 | 2460 |

| Mw | 2930 | 2931 | 2932 | 2933 | 2934 | 2935 | 2936 | 2937 | 2938 | 2939 | 2940 | 2941 | 2942 | 2943 | 2944 | 2945 | 2946 | 2947 | 2948 | 2949 | 2950 | 2951 | 2952 | 2953 | 2954 | 2955 | 2956 | 2957 | 2958 | 2959 | 2960 | 2961 | 2962 | 2963 | 2964 | 2965 | 2966 | 2967 | 2968 | 2969 |
|---|---|---|---|---|---|---|---|---|---|---|---|---|---|---|---|---|---|---|---|---|---|---|---|---|---|---|---|---|---|---|---|---|---|---|---|---|---|---|---|---|
| Intensity | 1990 | 1990 | 2110 | 2020 | 1480 | 2420 | 2690 | 2370 | 2370 | 2270 | 3100 | 2950 | 2290 | 2910 | 2830 | 2030 | 2240 | 3540 | 2540 | 2620 | 2100 | 2360 | 2490 | 1860 | 2420 | 2850 | 2170 | 3020 | 2630 | 2940 | 2180 | 2160 | 2300 | 1960 | 2170 | 2660 | 2200 | 2300 | 1860 | 1540 |

| Mw | 2970 | 2971 | 2972 | 2973 | 2974 | 2975 | 2976 | 2977 | 2978 | 2979 | 2980 | 2981 | 2982 | 2983 | 2984 | 2985 | 2986 | 2987 | 2988 | 2989 | 2990 | 2991 | 2992 | 2993 | 2994 | 2995 | 2996 | 2997 | 2998 | 2999 |
|---|---|---|---|---|---|---|---|---|---|---|---|---|---|---|---|---|---|---|---|---|---|---|---|---|---|---|---|---|---|---|
| Intensity | 2600 | 1900 | 2950 | 1920 | 3150 | 2590 | 2580 | 1800 | 2240 | 2020 | 2490 | 2900 | 2280 | 2610 | 2660 | 2210 | 2650 | 2570 | 2410 | 2200 | 2480 | 2320 | 2400 | 2820 | 2400 | 2290 | 1760 | 2000 | 2500 | 1790 |

(b) The theoretical molecular weights of the monoester form, dehydration product of the monoester form, diester form, dehydration product of the diester form, triester form, and dehydration product of the triester form of the polyglycerol fatty acid ester, as well as the theoretical molecular weights of the polyglycerol and the dehydration product of the polyglycerol, are calculated. Note that the theoretical molecular weights of the monoester form, the diester form, the triester form, and the polyglycerol are those when the degree of polymerization of glycerol is from 1 to 20. The theoretical molecular weights of the dehydration product of the monoester form, the dehydration product of the diester form, the dehydration product of the triester form, and the dehydration product of the polyglycerol are those when the degree of polymerization of glycerol is from 1 to 20 and when the degree of dehydration is from 1 to 3 (from 1 to 3 water molecules are removed).

Hereinafter, (b) in Example 1 will be described.

In Example 1, the theoretical molecular weights of the monoester form of the polyglycerol monocaprate are shown in the section of "Monoester form" in Table 7. The theoretical molecular weights of the dehydration products of the monoester form are shown in the sections of "Dehydration product of monoester form (degree of dehydration = 1)", "Dehydration product of monoester form (degree of dehydration = 2)", and "Dehydration product of monoester form (degree of dehydration = 3)" in Table 7. The same applies to the diester form and the like that are other constituent components.

[Table 7]

Table 7

| | Degree of Polymerization of Glycerol | | | | | | | | | | | | | | | | | | | |
|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 |
| Monoester Form | 246.34 | 320.42 | 394.50 | 468.58 | 542.66 | 616.74 | 690.81 | 764.89 | 838.97 | 913.05 | 987.13 | 1061.21 | 1135.29 | 1209.36 | 1283.44 | 1357.52 | 1431.60 | 1505.68 | 1579.76 | 1653.83 |
| Dehydration Product of Monoester Form (Degree of Dehydration = 1) | 228.33 | 302.41 | 376.49 | 450.56 | 524.64 | 598.72 | 672.80 | 746.88 | 820.96 | 895.03 | 969.11 | 1043.19 | 1117.27 | 1191.35 | 1265.43 | 1339.51 | 1413.58 | 1487.66 | 1561.74 | 1635.82 |
| Dehydration Product of Monoester Form (Degree of Dehydration = 2) | 210.31 | 284.39 | 358.47 | 432.55 | 506.63 | 580.71 | 654.78 | 728.86 | 802.94 | 877.02 | 951.10 | 1025.18 | 1099.25 | 1173.33 | 1247.41 | 1321.49 | 1395.57 | 1469.65 | 1543.73 | 1617.80 |
| Dehydration Product of Monoester Form (Degree of Dehydration = 3) | 192.30 | 266.38 | 340.45 | 414.53 | 488.61 | 562.69 | 636.77 | 710.85 | 784.93 | 859.00 | 933.08 | 1007.16 | 1081.24 | 1155.32 | 1229.40 | 1303.47 | 1377.55 | 1451.63 | 1525.71 | 1599.79 |
| Diester Form | 400.59 | 474.67 | 548.75 | 622.83 | 696.91 | 770.99 | 845.06 | 919.14 | 993.22 | 1067.30 | 1141.38 | 1215.46 | 1289.53 | 1363.61 | 1437.69 | 1511.77 | 1585.86 | 1659.93 | 1734.01 | 1808.08 |
| Dehydration Product of Diester Form (Degree of Dehydration = 1) | 382.58 | 456.66 | 530.73 | 604.81 | 678.89 | 752.97 | 827.05 | 901.13 | 975.21 | 1049.28 | 1123.36 | 1197.44 | 1271.52 | 1345.60 | 1419.68 | 1493.76 | 1567.83 | 1641.91 | 1715.99 | 1790.07 |
| Dehydration Product of Diester Form (Degree of Dehydration = 2) | 364.56 | 438.64 | 512.72 | 586.80 | 660.88 | 734.95 | 809.03 | 883.11 | 957.19 | 1031.27 | 1105.35 | 1179.43 | 1253.50 | 1327.58 | 1401.66 | 1475.74 | 1549.82 | 1623.90 | 1697.98 | 1772.05 |
| Dehydration Product of Diester Form (Degree of Dehydration = 3) | 346.55 | 420.63 | 494.70 | 558.78 | 642.86 | 716.94 | 791.02 | 865.10 | 939.17 | 1013.25 | 1087.33 | 1161.41 | 1235.49 | 1309.57 | 1383.65 | 1457.72 | 1531.80 | 1605.88 | 1679.96 | 1754.04 |
| Triester Form | 554.84 | 628.92 | 703.00 | 777.08 | 851.16 | 925.23 | 999.31 | 1073.39 | 1147.47 | 1221.55 | 1295.63 | 1369.71 | 1443.78 | 1517.86 | 1591.94 | 1666.02 | 1740.10 | 1814.18 | 1888.26 | 1962.33 |
| Dehydration Product of Triester Form (Degree of Dehydration = 1) | 536.83 | 610.91 | 684.98 | 759.06 | 833.14 | 907.22 | 981.30 | 1055.38 | 1129.46 | 1203.53 | 1277.61 | 1351.69 | 1425.77 | 1499.85 | 1573.93 | 1648.00 | 1722.08 | 1796.16 | 1870.24 | 1944.32 |
| Dehydration Product of Triester Form (Degree of Dehydration = 2) | 518.81 | 592.89 | 666.97 | 741.05 | 815.13 | 889.20 | 963.28 | 1037.36 | 1111.44 | 1185.52 | 1259.60 | 1333.68 | 1407.76 | 1481.83 | 1555.91 | 1629.99 | 1704.07 | 1778.15 | 1852.22 | 1926.30 |
| Dehydration Product of Triester Form (Degree of Dehydration = 3) | 500.80 | 574.87 | 648.95 | 723.03 | 797.11 | 871.19 | 945.27 | 1019.35 | 1093.42 | 1167.50 | 1241.58 | 1315.66 | 1389.74 | 1463.82 | 1537.90 | 1611.97 | 1686.05 | 1760.13 | 1834.21 | 1908.29 |
| Polyglycerol | 92.09 | 166.17 | 240.25 | 314.33 | 388.41 | 462.49 | 536.56 | 610.64 | 684.72 | 758.80 | 832.88 | 906.96 | 981.04 | 1055.11 | 1129.19 | 1203.27 | 1277.35 | 1351.43 | 1425.51 | 1499.59 |
| Dehydration Product of Polyglycerol (Degree of Dehydration = 1) | 74.08 | 148.16 | 222.24 | 296.31 | 370.39 | 444.47 | 518.55 | 592.63 | 666.71 | 740.79 | 814.86 | 888.94 | 963.02 | 1037.10 | 1111.18 | 1185.26 | 1259.33 | 1333.41 | 1407.49 | 1481.57 |
| Dehydration Product of Polyglycerol (Degree of Dehydration = 2) | 56.06 | 130.14 | 204.22 | 278.30 | 352.38 | 426.46 | 500.53 | 574.61 | 648.69 | 722.77 | 796.85 | 870.93 | 945.01 | 1019.08 | 1093.16 | 1167.24 | 1241.32 | 1315.40 | 1389.48 | 1463.55 |
| Dehydration Product of Polyglycerol (Degree of Dehydration = 3) | 38.05 | 112.13 | 186.20 | 260.28 | 334.36 | 408.44 | 482.52 | 556.60 | 630.68 | 704.75 | 778.83 | 852.91 | 926.99 | 1001.07 | 1075.15 | 1149.23 | 1223.30 | 1297.38 | 1371.46 | 1445.54 |

(c) In order to be made to correspond to each molecular weight obtained in (a) by the time-of-flight mass spectrometer, the theoretical molecular weight in (b) was corrected by the following method to obtain a "corrected molecular weight".

• Correction Method

The component whose molecular weight is measured and detected by the time-of-flight mass spectrometer is a molecule [M-H]⁻ deprotonated by ionization. Therefore, the molecular weight obtained by rounding down the theoretical molecular weight in (b) to an integer is subtracted by 1, which corresponds to each molecular weight obtained in (a). However, when the molecular weight is large, the molecular weight at the peak top of the intensity may be shifted by 1 due to an error or the like in the molecular weight calculation or the rounding down of the measured molecular weight to an integer. Therefore, with regard to those having a theoretical molecular weight of 700 or more in (b), the peak intensities corresponding to three points of a molecular weight (M1) obtained by rounding down the theoretical molecular weight in (b) to an integer, a molecular weight (M2) obtained by subtracting 1 from M1, and a molecular weight (M3) obtained by subtracting 2 from M1, were compared, and the molecular weight having the highest peak intensity among them was defined as the "corrected molecular weight". In addition, with respect to those having a theoretical molecular weight of less than 700 in (b), a molecular weight obtained by subtracting 1 from the molecular weight obtained by rounding down the theoretical molecular weight in (b) to an integer was defined as the "corrected molecular weight".

• Adjustment After Correction

Even in different structures, their corrected molecular weights obtained by the above method may be the same depending on the degree of dehydration of the polyglycerol chain, the degree of polymerization of the polyglycerol chain, and the number of bonds (esterification number) of the polyglycerol chain to the fatty acid. Based on the molecular design of the polyglycerol fatty acid ester, the content of that having a larger esterification number or that having a higher degree of dehydration becomes smaller, and thus, among molecules having the same corrected molecular weight, those other than the highest-ranked molecule according to the following criteria were not included in the peak intensity.

• Concept of Priority

1. Esterification number: small > large
2. Degree of dehydration: low > high
3. In the above criteria 1 and 2, when the rankings of the comparison targets are different, the rankings are determined in the order of 1 > 2. For example, in comparison between specific components (A) and (B), when 1. the component (B) has a smaller esterification number and 2. the component (A) has a smaller degree of dehydration of glycerol, 1. the component (B) having a smaller esterification number is prioritized because 1 > 2 in the above criteria.

In addition, in consideration of the esterification number and degree of polymerization of glycerol of the polyglycerol fatty acid ester, a polyglycerol fatty acid ester having a degree of dehydration that cannot exist is not included in the peak intensity. For example, in order to remove one water molecule from the polyglycerol fatty acid ester (to set the degree of dehydration to 1), the polyglycerol fatty acid ester needs to have two or more hydroxyl groups. Therefore, the polyglycerol fatty acid ester having only one hydroxyl group does not have a degree of dehydration of from 1 to 3.

Hereinafter, (c) in Example 1 will be described.

For each component of the polyglycerol monocaprate calculated in Table 7, with regard to those having a theoretical molecular weight of less than 700 in (b), the theoretical molecular weight was rounded down to an integer, and 1 was subtracted from the integer to obtain the "corrected molecular weight". For example, the monoester form (degree of polymerization of glycerol = 4) has a theoretical molecular weight of 468.58, and the corrected molecular weight is 467.

With regard to those having a theoretical molecular weight of 700 or more in (b), the peak intensities corresponding to three points of a molecular weight (M1) obtained by rounding down the theoretical molecular weight in (b) to an integer, a molecular weight (M2) obtained by subtracting 1 from M1, and a molecular weight (M3) obtained by subtracting 2 from M1, were compared, and the molecular weight having the highest intensity among them was defined as the "corrected molecular weight". For example, the theoretical molecular weight of the polyglycerol (degree of polymerization of glycerol = 11, degree of dehydration = 3) is 778.83, and thus the molecular weight (M1) is 778, the molecular weight (M2) is 777, and the molecular weight (M3) is 776. The peak intensity corresponding to the molecular weight (M1) is 6780, the peak intensity corresponding to the molecular weight (M2) is 8510, and the peak intensity corresponding to the molecular weight (M3) is 6740 (see Table 2). Therefore, 8510 that is the largest peak intensity, that is, 777 = molecular weight (M2) is the "corrected molecular weight".

For example, the theoretical molecular weight of the triester form (degree of polymerization of glycerol = 4) is 777.8, and thus the molecular weight (M1) is 777, the molecular weight (M2) is 776, and the molecular weight (M3) is 775. The peak intensity corresponding to the molecular weight (M1) is 8510, the peak intensity corresponding to the molecular weight (M2) is 6740, and the peak intensity corresponding to the molecular weight (M3) is 7840 (see Table 2). Therefore, 8510 that is the largest peak intensity, that is, 777 = molecular weight (M1) can be the "corrected molecular weight". However, since the corrected molecular weight is the same value as that of the above polyglycerol (degree of polymerization of glycerol = 11, degree of dehydration = 3), the polyglycerol (degree of polymerization of glycerol = 11, degree of dehydration = 3) has a corrected molecular weight of "777" according to the above "Concept of Priority", and the triester form (degree of polymerization of glycerol = 4) is not included in the peak intensity.

In addition, the monoester form (degree of polymerization of glycerol = 1) has two hydroxyl groups, and is included in the peak intensity when the degree of dehydration is 1 because it exists, but is not included in the peak intensity when the degree of dehydration is 2 or 3 because it does not exist. Since the diester form (degree of polymerization of glycerol = 1) has only one hydroxyl group, diester forms having a degree of dehydration of from 1 to 3 do not exist, and are not included in the peak intensity. In consideration of the above, the corrected molecular weights of the respective components are summarized in Table 8. "-" indicates a component not included in the peak intensity.

[Table 8]

Table 8

| | Degree of Polymerization of Glycerol | | | | | | | | | | | | | | | | | | | |
|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 |
| Monoester Form | 245 | 319 | 393 | 467 | 541 | 615 | 689 | 763 | 837 | 911 | 985 | 1059 | 1133 | 1207 | 1281 | 1355 | 1429 | 1503 | 1579 | 1651 |
| Dehydration Product of Monoester Form (Degree of Dehydration = 1) | 227 | 301 | 375 | 449 | 523 | 597 | 671 | 745 | 819 | 893 | 967 | 1041 | 1115 | 1189 | 1265 | 1337 | 1413 | 1487 | 1560 | 1635 |
| Dehydration Product of Monoester Form (Degree of Dehydration = 2) | - | - | 357 | 431 | 505 | 579 | 653 | 727 | 801 | 875 | 949 | 1023 | 1097 | 1171 | 1245 | 1321 | 1395 | 1467 | 1543 | 1615 |
| Dehydration Product of Monoester Form (Degree of Dehydration = 3) | - | - | - | - | 487 | 561 | 635 | 709 | 783 | 859 | 933 | 1007 | 1081 | 1155 | 1229 | 1303 | 1377 | 1449 | 1525 | 1599 |
| Diester | 399 | 473 | 547 | 621 | 695 | 769 | 845 | 919 | 993 | 1065 | 1141 | 1215 | 1289 | 1363 | 1435 | 1510 | 1585 | 1658 | 1734 | 1808 |
| Dehydration Product of Diester Form (Degree of Dehydration = 1) | - | 455 | 529 | 603 | 677 | 751 | 825 | 899 | 973 | 1047 | 1121 | 1195 | 1271 | 1343 | 1417 | 1493 | 1565 | 1641 | 1715 | 1789 |
| Dehydration Product of Diester Form (Degree of Dehydration = 2) | - | - | - | 585 | 659 | 732 | 809 | 883 | 957 | 1031 | 1105 | 1179 | 1253 | 1326 | 1401 | 1474 | 1548 | 1621 | 1697 | 1771 |
| Dehydration Product of Diester Form (Degree of Dehydration = 3) | - | - | - | - | - | 715 | 789 | 863 | 937 | 1011 | 1085 | 1159 | 1235 | 1308 | 1381 | 1455 | 1529 | 1605 | 1677 | 1752 |
| Triester Form | 553 | 627 | 701 | - | - | - | 998 | 1071 | 1145 | 1219 | - | 1368 | 1442 | 1515 | 1591 | 1654 | 1740 | 1813 | 1888 | 1961 |
| Dehydration Product of Triester Form (Degree of Dehydration = 1) | - | - | - | - | - | - | - | - | - | - | - | - | - | - | 1573 | 1646 | 1721 | 1794 | 1869 | 1943 |
| Dehydration Product of Triester Form (Degree of Dehydration = 2) | - | - | - | - | - | - | - | - | - | - | - | - | - | - | 1553 | 1629 | 1702 | 1776 | 1852 | 1925 |
| Dehydration Product of Triester Form (Degree of Dehydration = 3) | - | - | - | - | - | - | - | - | - | - | - | - | - | - | 1535 | 1610 | 1685 | 1758 | 1834 | 1906 |
| Polyglycerol | 91 | 165 | 239 | 313 | 387 | 461 | 535 | 609 | 683 | 757 | 831 | 905 | 979 | 1053 | 1127 | 1201 | 1275 | 1350 | 1424 | 1499 |
| Dehydration Product of Polyglycerol (Degree of Dehydration = 1) | 73 | 147 | 221 | 295 | 369 | 443 | 517 | 591 | 665 | 739 | 813 | 887 | 961 | 1035 | 1109 | 1183 | 1257 | 1333 | 1405 | 1480 |
| Dehydration Product of Polyglycerol (Degree of Dehydration = 2) | - | 129 | 203 | 277 | 351 | 425 | 499 | 573 | 647 | 721 | 795 | 869 | 943 | 1017 | 1091 | 1165 | 1239 | 1313 | 1388 | 1461 |
| Dehydration Product of Polyglycerol (Degree of Dehydration = 3) | - | - | - | 259 | 333 | 407 | 481 | 555 | 629 | 703 | 777 | 851 | 925 | 1001 | 1075 | 1147 | 1221 | 1295 | 1369 | 1444 |

(d) The intensities obtained in (a) are made to correspond to the corrected molecular weights of the respective components of the polyglycerol mono-fatty acid ester product calculated in (c), and these are added up to obtain the peak intensities of the "monoester form", "dehydration product of the monoester form", "diester form", "dehydration product of the diester form", "triester form", "dehydration product of the triester form", "polyglycerol", and "dehydration product of the polyglycerol" specified in P1 to 3.

Hereinafter, (d) in Example 1 will be described.

Table 9 shows the intensities obtained in (a) that are made to correspond to the corrected molecular weights of the respective components of the polyglycerol monocaprate calculated in (b). Based on Table 9, the peak intensities of the "monoester form", "dehydration product of the monoester form", "diester form", "dehydration product of the diester form", "triester form", "dehydration product of the triester form", "polyglycerol", and "dehydration product of the polyglycerol" specified in P1 will be described.

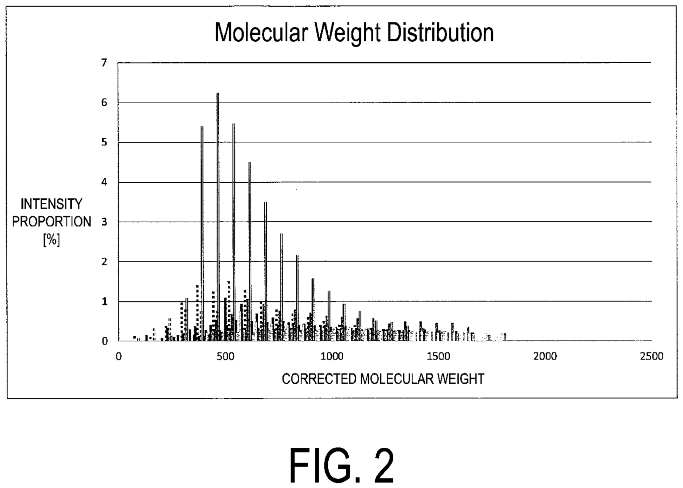

FIG. 2 is a chart of the corrected molecular weight and the corresponding peak intensity proportion (%) in the polyglycerol monocaprate of Example 1. The hollow bar indicates the monoester form.

Table 9

| | Degree of Polymerization of Glycerol | | | | | | | | | | | | | | | | | | | | Total |
|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 | |
| Monoester Form | 4730 | 37500 | 187000 | 217000 | 190000 | 156000 | 121000 | 94000 | 74900 | 54500 | 43600 | 32200 | 26500 | 17800 | 16600 | 13200 | 11300 | 9090 | 8600 | 8800 | 1324420 |
| Dehydration Product of Monoester Form (Degree of Dehydration = 1) | 11400 | 6730 | 4110 | 8020 | 10800 | 9230 | 10900 | 14200 | 15900 | 15600 | 14400 | 11200 | 11400 | 10800 | 10200 | 9630 | 8790 | 8650 | 8660 | 7210 | 207830 |
| Dehydration Product of Monoester Form (Degree of Dehydration = 2) | - | - | 12900 | 14500 | 14100 | 11600 | 11400 | 10500 | 11200 | 10700 | 9570 | 10800 | 9270 | 9550 | 10400 | 10100 | 8120 | 7950 | 7500 | 8210 | 188370 |
| Dehydration Product of Monoester Form (Degree of Dehydration = 3) | - | - | - | - | 6930 | 7760 | 7260 | 9540 | 9990 | 14700 | 14300 | 12700 | 12000 | 10400 | 11000 | 10000 | 8560 | 8290 | 8440 | 7230 | 159100 |
| Diester Form | 4590 | 9240 | 18400 | 17700 | 16700 | 17600 | 18900 | 16900 | 15100 | 13600 | 12100 | 11600 | 10800 | 9770 | 9970 | 8690 | 8920 | 8490 | 7330 | 7600 | 244000 |
| Dehydration Product of Diester Form (Degree of Dehydration = 1) | - | 9520 | 11700 | 18500 | 16400 | 13400 | 13900 | 12500 | 11200 | 10600 | 10000 | 9940 | 8080 | 8680 | 8610 | 8030 | 8020 | 7400 | 7180 | 7470 | 201130 |
| Dehydration Product of Diester Form (Degree of Dehydration = 2) | - | - | - | 10100 | 8890 | 8570 | 15500 | 14700 | 13200 | 12100 | 10300 | 9950 | 10600 | 9500 | 9190 | 7900 | 8000 | 7470 | 6590 | 6850 | 169410 |
| Dehydration Product of Diester Form (Degree of Dehydration = 3) | - | - | - | - | - | 15400 | 14200 | 14900 | 12400 | 12200 | 12600 | 11500 | 10400 | 9680 | 9040 | 8550 | 9160 | 7570 | 7220 | 7750 | 162570 |
| Triester Form | 7180 | 5610 | 7790 | - | - | - | 8850 | 9220 | 9150 | 8720 | - | 8130 | 8320 | 8130 | 7480 | 8120 | 6810 | 6660 | 6290 | 6440 | 122900 |
| Dehydration Product of Triester Form (Degree of Dehydration = 1) | - | - | - | - | - | - | - | - | - | - | - | - | - | - | 7180 | 7600 | 8130 | 6840 | 6580 | 6320 | 42650 |
| Dehydration Product of Triester Form (Degree of Dehydration = 2) | - | - | - | - | - | - | - | - | - | - | - | - | - | - | 8010 | 7780 | 7370 | 6940 | 6580 | 6450 | 43130 |
| Dehydration Product of Triester Form (Degree of Dehydration = 3) | - | - | - | - | - | - | - | - | - | - | - | - | - | - | 7980 | 7530 | 7430 | 7240 | 6740 | 5930 | 42850 |
| Polyglycerol | 2470 | 11000 | 19900 | 27800 | 26000 | 26700 | 24100 | 23600 | 21000 | 18900 | 16200 | 17100 | 13000 | 12000 | 10600 | 13000 | 10900 | 9860 | 8630 | 7640 | 320400 |
| Dehydration Product of Polyglycerol (Degree of Dehydration = 1) | 4340 | 3810 | 13200 | 33900 | 48800 | 43600 | 52400 | 44300 | 34300 | 27800 | 23900 | 21000 | 17700 | 14500 | 14000 | 11200 | 10200 | 9150 | 8680 | 8930 | 445710 |
| Dehydration Product of Polyglycerol (Degree of Dehydration = 2) | - | 5270 | 2630 | 5380 | 5670 | 5750 | 38100 | 32500 | 24500 | 20700 | 16400 | 15500 | 14400 | 12200 | 11400 | 10700 | 10300 | 9430 | 8860 | 8710 | 258400 |
| Dehydration Product of Polyglycerol (Degree of Dehydration = 3) | - | - | - | 3510 | 10100 | 9610 | 8030 | 7340 | 7230 | 8140 | 8510 | 9440 | 9140 | 9670 | 10300 | 8960 | 8700 | 8950 | 7900 | 8830 | 144360 |

[Table 9]

EP 4 722 189 A1

The peak intensity of the "monoester form" is a sum when the degrees of polymerization of glycerol of the monoester form in Table 9 are from 1 to 20, which is 1324420.

The peak intensity of the "diester form" is a sum when the degrees of polymerization of glycerol of the diester form in Table 9 are from 1 to 20, which is 244000.

The peak intensity of the "triester form" is a sum when the degrees of polymerization of glycerol of the triester form in Table 9 are from 1 to 20, which is 122900.

The peak intensity of the "polyglycerol" is a sum when the degrees of polymerization of glycerol of the polyglycerol in Table 9 are from 1 to 20, which is 320400.

The peak intensity of the "dehydration product of the monoester form" is a sum when the degrees of polymerization of glycerol of the dehydration product of the monoester form in Table 9 are from 1 to 20 and the degrees of dehydration are from 1 to 3, which is 555300 (= 207830 + 188370 + 159100).

The peak intensity of the "dehydration product of the diester form" is a sum when the degrees of polymerization of glycerol of the dehydration product of the diester form in Table 9 are from 1 to 20 and the degrees of dehydration are from 1 to 3, which is 533110 (= 201130 + 169410 + 162570).

The peak intensity of the "dehydration product of the triester form" is a sum when the degrees of polymerization of glycerol of the dehydration product of the triester form in Table 9 are from 1 to 20 and the degrees of dehydration are from 1 to 3, which is 128630 (= 42650 + 43130 + 42850).

The peak intensity of the "dehydration product of the polyglycerol" is a sum when the degrees of polymerization of glycerol of the dehydration product of the polyglycerol in Table 9 are from 1 to 20 and the degrees of dehydration are from 1 to 3, which is 848470 (= 445710 + 258400 + 144360).

(e) Formula (X) and Formula (Y) above are calculated using the peak intensities of the respective components of the polyglycerol mono-fatty acid ester product calculated in (d).

Hereinafter, (e) in Example 1 will be described.

$$\text{Formula (X)} = P2/P1 = 1324420/4077230 = 0.325$$

$$\text{Formula (Y)} = P3/P1 = (320400 + 848470)/4077230 = 0.287$$

Example 2

In a 1-liter four-neck flask equipped with a nitrogen-introducing tube, a stirrer, a condenser, a temperature controller, and a dropping cylinder, 0.55 mol (94.3 g) of capric acid was charged and heated to 120°C. Then, 5.48 mol (405.67 g) of glycidol was added dropwise over 10 hours while the reaction temperature was maintained at 120°C, and 0.004 mol (0.4 g) of 42.5% phosphoric acid was added dropwise during the dropwise addition of glycidol. Then, the mixture was aged for 3 hours while being maintained at 120°C. Then, the temperature was raised to 130°C, and the reaction was continued until the oxirane concentration in the system became less than 0.1%. After cooling, the reaction product was taken out to obtain about 500 g of polyglycerol monocaprate having an average degree of polymerization of glycerol of 10.

The obtained polyglycerol monocaprate was subjected to HPLC analysis, and it was found that the area ratio of peaks corresponding to a monoester form, a dehydration product of the monoester form, a polyester form, a dehydration product of the polyester form, as well as peaks corresponding to polyglycerol and a dehydration product of the polyglycerol, each of the peaks existing within a retention time of from 6.0 to 20.0 minutes in the HPLC spectrum, was 93.8%.

Furthermore, the obtained polyglycerol monocaprate was subjected to mass spectrometry in the same manner as in Example 1, and the peak intensity ratio (P2/P1) was calculated according to Formula (X) above, which was found to be 0.353. The peak intensity ratio (P3/P1) was calculated according to Formula (Y) above, which was found to be 0.322.

The HLB value of the obtained polyglycerol monocaprate was 16.8.

Example 3

In a 1-liter four-neck flask equipped with a nitrogen-introducing tube, a stirrer, a condenser, a temperature controller, and a dropping cylinder, 0.53 mol (106.4 g) of lauric acid was charged and heated to 120°C. Then, 5.31 mol (393.6 g) of glycidol was added dropwise over 10 hours while the reaction temperature was maintained at 120°C. During the dropwise addition of glycidol, 0.004 mol (0.4 g) of 42.5% phosphoric acid was added dropwise. Then, the mixture was aged for 3 hours while being maintained at 120°C. Then, the temperature was raised to 130°C, and the reaction was continued until the oxirane concentration in the system became less than 0.1%. After cooling, the reaction product was taken out to obtain about 500 g of polyglycerol monolaurate having an average degree of polymerization of glycerol of 10.

The obtained polyglycerol monolaurate was subjected to HPLC analysis, and the area ratio of peaks corresponding to a monoester form, a dehydration product of the monoester form, a polyester form, a dehydration product of the polyester form, as well as peaks corresponding to polyglycerol and a dehydration product of the polyglycerol, each of the peaks existing within a retention time of from 6.0 to 20.0 minutes in the HPLC spectrum, was 98.9%.

Furthermore, the obtained polyglycerol monolaurate was subjected to mass spectrometry in the same manner as in Example 1, and the peak intensity ratio (P2/P1) was calculated according to Formula (X) above, which was found to be 0.429. The peak intensity ratio (P3/P1) was calculated according to Formula (Y) above, which was found to be 0.348.

The HLB value of the obtained polyglycerol monolaurate was 16.0.

Example 4

In a 1-liter four-neck flask equipped with a nitrogen-introducing tube, a stirrer, a condenser, a temperature controller, and a dropping cylinder, 0.74 mol (169.7 g) of myristic acid was charged and heated to 120°C. Then, 4.46 mol (330.3 g) of glycidol was added dropwise over 10 hours while the reaction temperature was maintained at 120°C, and 0.004 mol (0.4 g) of 42.5% phosphoric acid was added dropwise during the dropwise addition of glycidol. Then, the mixture was aged for 3 hours while being maintained at 120°C. Then, the temperature was raised to 130°C, and the reaction was continued until the oxirane concentration in the system became less than 0.1%. After cooling, the reaction product was taken out to obtain about 500 g of polyglycerol monomyristate having an average degree of polymerization of glycerol of 6.

The obtained polyglycerol monomyristate was subjected to HPLC analysis, and it was found that the area ratio of peaks corresponding to a monoester form, a dehydration product of the monoester form, a polyester form, and a dehydration product of the polyester form, as well as peaks corresponding to polyglycerol and a dehydration product of the polyglycerol, each of the peaks existing within a retention time of from 6.0 to 20.0 minutes in the HPLC spectrum, was 96.2%.

Furthermore, the obtained polyglycerol monomyristate was subjected to mass spectrometry in the same manner as in Example 1, and the peak intensity ratio (P2/P1) was calculated according to Formula (X) above, which was found to be 0.446. The peak intensity ratio (P3/P1) was calculated according to Formula (Y) above, which was found to be 0.267.

The HLB value of the obtained polyglycerol monomyristate was 13.4.

Example 5

In a 1-liter four-neck flask equipped with a nitrogen-introducing tube, a stirrer, a condenser, a temperature controller, and a dropping cylinder, 0.52 mol (117.8 g) of myristic acid was charged and heated to 120°C. Then, 5.16 mol (382.18 g) of glycidol was added dropwise over 10 hours while the reaction temperature was maintained at 120°C, and 0.004 mol (0.4 g) of 42.5% phosphoric acid was added dropwise during the dropwise addition of glycidol. Then, the mixture was aged for 3 hours while being maintained at 120°C. Then, the temperature was raised to 130°C, and the reaction was continued until the oxirane concentration in the system became less than 0.1%. After cooling, the reaction product was taken out to obtain about 500 g of polyglycerol monomyristate having an average degree of polymerization of glycerol of 10.

The obtained polyglycerol monomyristate was subjected to HPLC analysis, and it was found that the area ratio of peaks corresponding to a monoester form, a dehydration product of the monoester form, a polyester form, and a dehydration product of the polyester form, as well as peaks corresponding to polyglycerol and a dehydration product of the polyglycerol, each of the peaks existing within a retention time of from 6.0 to 20.0 minutes in the HPLC spectrum, was 97.4%.

Furthermore, the obtained polyglycerol monomyristate was subjected to mass spectrometry in the same manner as in Example 1, and the peak intensity ratio (P2/P1) was calculated according to Formula (X) above, which was found to be 0.391. The peak intensity ratio (P3/P1) was calculated according to Formula (Y) above, which was found to be 0.314.

The HLB value of the obtained polyglycerol monomyristate was 15.2.

Example 6

In a 1-liter four-neck flask equipped with a nitrogen-introducing tube, a stirrer, a condenser, a temperature controller, and a dropping cylinder, 0.69 mol (195.1 g) of stearic acid was charged and heated to 120°C. Then, 4.12 mol (304.9 g) of glycidol was added dropwise over 10 hours while the reaction temperature was maintained at 120°C, and 0.004 mol (0.4 g) of 42.5% phosphoric acid was added dropwise during the dropwise addition of glycidol. Then, the mixture was aged for 3 hours while being maintained at 120°C. Then, the temperature was raised to 130°C, and the reaction was continued until the oxirane concentration in the system became less than 0.1%. After cooling, the reaction product was taken out to obtain about 500 g of polyglycerol monostearate having an average degree of polymerization of glycerol of 6.

The obtained polyglycerol monostearate was not soluble in the measurement solvent (10% methanol solution), and HPLC analysis was not able to be performed. However, in consideration of the tendency of the polyglycerol mono-fatty acid ester product in the production method and other Examples, it is presumed that the area ratio of the peaks corresponding to the monoester form, the dehydration product of the monoester form, the polyester form, and the dehydration product of the polyester form, as well as the peaks corresponding to the polyglycerol and the dehydration product of the polyglycerol, each of the peaks existing within a retention time of from 6.0 to 20.0 minutes in the HPLC spectrum, is at least 80% or more.

Furthermore, the obtained polyglycerol monostearate was subjected to mass spectrometry in the same manner as in Example 1, and the peak intensity ratio (P2/P1) was calculated according to Formula (X) above, which was found to be 0.256. The peak intensity ratio (P3/P1) was calculated according to Formula (Y) above, which was found to be 0.457.

The HLB value of the obtained polyglycerol monostearate was 11.9.

Example 7

In a 1-liter four-neck flask equipped with a nitrogen-introducing tube, a stirrer, a condenser, a temperature controller, and a dropping cylinder, 0.49 mol (138.7 g) of stearic acid was charged and heated to 120°C. Then, 4.88 mol (361.3 g) of glycidol was added dropwise over 10 hours while the reaction temperature was maintained at 120°C, and 0.004 mol (0.4 g) of 42.5% phosphoric acid was added dropwise during the dropwise addition of glycidol. Then, the mixture was aged for 3 hours while being maintained at 120°C. Then, the temperature was raised to 130°C, and the reaction was continued until the oxirane concentration in the system became less than 0.1%. After cooling, the reaction product was taken out to obtain about 500 g of polyglycerol monostearate having an average degree of polymerization of glycerol of 10.

The obtained polyglycerol monostearate was not soluble in the measurement solvent (10% methanol solution), and HPLC analysis was not able to be performed. However, in consideration of the tendency of the polyglycerol mono-fatty acid ester product in the production method and other Examples, it is presumed that the area ratio of the peaks corresponding to the monoester form, the dehydration product of the monoester form, the polyester form, and the dehydration product of the polyester form, as well as the peaks corresponding to the polyglycerol and the dehydration product of the polyglycerol, each of the peaks existing within a retention time of from 6.0 to 20.0 minutes in the HPLC spectrum, is at least 80% or more.

Furthermore, the obtained polyglycerol monostearate was subjected to mass spectrometry in the same manner as in Example 1, and the peak intensity ratio (P2/P1) was calculated according to Formula (X) above, which was found to be 0.410. The peak intensity ratio (P3/P1) was calculated according to Formula (Y) above, which was found to be 0.414.

The HLB value of the obtained polyglycerol monostearate was 14.0.

Example 8

In a 1-liter four-neck flask equipped with a nitrogen-introducing tube, a stirrer, a condenser, a temperature controller, and a dropping cylinder, 0.86 mol (244.0 g) of oleic acid was charged and heated to 120°C. Then, 3.46 mol (256.0 g) of glycidol was added dropwise over 20 hours while the reaction temperature was maintained at 120°C. Then, the mixture was aged for 3 hours while being maintained at 120°C. Then, the temperature was raised to 130°C, and the reaction was continued until the oxirane concentration in the system became less than 0.1%. After cooling, the reaction product was taken out to obtain about 500 g of polyglycerol monooleate having an average degree of polymerization of glycerol of 4.

The obtained polyglycerol monooleate was subjected to HPLC analysis, and it was found that the area ratio of peaks corresponding to a monoester form, a dehydration product of the monoester form, a polyester form, and a dehydration product of the polyester form, as well as peaks corresponding to polyglycerol and a dehydration product of the polyglycerol, each of the peaks existing within a retention time of from 6.0 to 20.0 minutes in the HPLC spectrum, was 95.5%.

Furthermore, the obtained polyglycerol monooleate was subjected to mass spectrometry in the same manner as in Example 1, and the peak intensity ratio (P2/P1) was calculated according to Formula (X) above, which was found to be 0.502. The peak intensity ratio (P3/P1) was calculated according to Formula (Y) above, which was found to be 0.316.

The HLB value of the obtained polyglycerol monooleate was 10.4.

Example 9

In a 1-liter four-neck flask equipped with a nitrogen-introducing tube, a stirrer, a condenser, a temperature controller, and a dropping cylinder, 0.69 mol (194.3 g) of oleic acid was charged and heated to 120°C. Then, 4.13 mol (305.7 g) of glycidol was added dropwise over 10 hours while the reaction temperature was maintained at 120°C, and 0.004 mol (0.4 g) of 42.5% phosphoric acid was added dropwise during the dropwise addition of glycidol. Then, the mixture was aged for 3 hours while being maintained at 120°C. Then, the temperature was raised to 130°C, and the reaction was continued until the oxirane concentration in the system became less than 0.1%. After cooling, the reaction product was taken out to obtain about 500 g of polyglycerol monooleate having an average degree of polymerization of glycerol of 6.

The obtained polyglycerol monooleate was subjected to HPLC analysis, and it was found that the area ratio of peaks corresponding to a monoester form, a dehydration product of the monoester form, a polyester form, and a dehydration product of the polyester form, as well as peaks corresponding to polyglycerol and a dehydration product of the polyglycerol, each of the peaks existing within a retention time of from 6.0 to 20.0 minutes in the HPLC spectrum, was 95.8%.

Furthermore, the obtained polyglycerol monooleate was subjected to mass spectrometry in the same manner as in Example 1, and the peak intensity ratio (P2/P1) was calculated according to Formula (X) above, which was found to be 0.558. The peak intensity ratio (P3/P1) was calculated according to Formula (Y) above, which was found to be 0.382.

The HLB value of the obtained polyglycerol monooleate was 12.0.

Example 10

In a 1-liter four-neck flask equipped with a nitrogen-introducing tube, a stirrer, a condenser, a temperature controller, and a dropping cylinder, 0.49 mol (138.0 g) of oleic acid was charged and heated to 120°C. Then, 4.89 mol (362.0 g) of glycidol was added dropwise over 10 hours while the reaction temperature was maintained at 120°C, and 0.004 mol (0.4 g) of 42.5% phosphoric acid was added dropwise during the dropwise addition of glycidol. Then, the mixture was aged for 3 hours while being maintained at 120°C. Then, the temperature was raised to 130°C, and the reaction was continued until the oxirane concentration in the system became less than 0.1%. After cooling, the reaction product was taken out to obtain about 500 g of polyglycerol monooleate having an average degree of polymerization of glycerol of 10.

The obtained polyglycerol monooleate was subjected to HPLC analysis, and it was found that the area ratio of peaks corresponding to a monoester form, a dehydration product of the monoester form, a polyester form, and a dehydration product of the polyester form, as well as peaks corresponding to polyglycerol and a dehydration product of the polyglycerol, each of the peaks existing within a retention time of from 6.0 to 20.0 minutes in the HPLC spectrum, was 98.6%.

Furthermore, the obtained polyglycerol monooleate was subjected to mass spectrometry in the same manner as in Example 1, and the peak intensity ratio (P2/P1) was calculated according to Formula (X) above, which was found to be 0.274. The peak intensity ratio (P3/P1) was calculated according to Formula (Y) above, which was found to be 0.435.

The HLB value of the obtained polyglycerol monooleate was 14.0.

Example 11

In a 1-liter four-neck flask equipped with a nitrogen-introducing tube, a stirrer, a condenser, a temperature controller, and a dropping cylinder, 0.86 mol (244.9 g) of isostearic acid was charged and heated to 120°C. Then, 3.44 mol (255.1 g) of glycidol was added dropwise over 20 hours while the reaction temperature was maintained at 120°C. Then, the mixture was aged for 3 hours while being maintained at 120°C. Then, the temperature was raised to 130°C, and the reaction was continued until the oxirane concentration in the system became less than 0.1%. After cooling, the reaction product was taken out to obtain about 500 g of polyglycerol monoisostearate having an average degree of polymerization of glycerol of 4.

The obtained polyglycerol monoisostearate was subjected to HPLC analysis, and it was found that the area ratio of peaks corresponding to a monoester form, a dehydration product of the monoester form, a polyester form, and a dehydration product of the polyester form, as well as peaks corresponding to polyglycerol and a dehydration product of the polyglycerol, each of the peaks existing within a retention time of from 6.0 to 20.0 minutes in the HPLC spectrum, was 97.6%.

Furthermore, the obtained polyglycerol monoisostearate was subjected to mass spectrometry in the same manner as in Example 1, and the peak intensity ratio (P2/P1) was calculated according to Formula (X) above, which was found to be 0.507. The peak intensity ratio (P3/P1) was calculated according to Formula (Y) above, which was found to be 0.307.

The HLB value of the obtained polyglycerol monoisostearate was 10.5.

Comparative Example 1

SY Glyster MO-7S (polyglycerol monooleate having an average degree of polymerization of glycerol of 10, available from Sakamoto Yakuhin Kogyo Co., Ltd) was subjected to HPLC analysis, and it was found that the area ratio of peaks corresponding to a monoester form, a dehydration product of the monoester form, a polyester form, and a dehydration product of the polyester form, as well as peaks corresponding to polyglycerol and a dehydration product of the polyglycerol, each of the peaks existing within a retention time of from 6.0 to 20.0 minutes in the HPLC spectrum, was 95.5%. In addition, mass spectrometry was performed in the same manner as in Example 1, and the peak intensity ratio (P2/P1) was calculated according to Formula (X) above, which was found to be 0.167. The peak intensity ratio (P3/P1) was calculated according to Formula (Y) above, which was found to be 0.447.

The HLB value of the SY Glyster MO-7S was 14.0.

Comparative Example 2

In a 1-liter four-neck flask equipped with a nitrogen-introducing tube, a stirrer, a condenser, a temperature controller, and a dropping cylinder, 0.5 mol (100.1 g) of lauric acid and 0.118 g of 42.5% phosphoric acid were charged and heated to 140°C. Then, 5.0 mol (370.40) of glycidol was added dropwise over 5 hours while the reaction temperature was maintained at 140°C, and the reaction was continued until the oxirane concentration in the system became less than 0.1%. After cooling, the reaction product was taken out to obtain about 470 g of polyglycerol monolaurate having an average degree of polymerization of glycerol of 10.

The obtained polyglycerol monolaurate was subjected to HPLC analysis, and it was found that the area ratio of peaks corresponding to a monoester form, a dehydration product of the monoester form, a polyester form, and a dehydration product of the polyester form, as well as peaks corresponding to polyglycerol and a dehydration product of the polyglycerol, each of the peaks existing within a retention time of from 6.0 to 20.0 minutes in the HPLC spectrum, was 98.4%.

Furthermore, the obtained polyglycerol monolaurate was subjected to mass spectrometry in the same manner as in Example 1, and the peak intensity ratio (P2/P1) was calculated according to Formula (X) above, which was found to be 0.410. The peak intensity ratio (P3/P1) was calculated according to Formula (Y) above, which was found to be 0.496.

The HLB value of the polyglycerol monolaurate was 16.0.

Evaluation

Sample 1 (cosmetic composition) containing the polyglycerol monooleate having an average degree of polymerization of glycerol of 4 that was obtained in Example 8, the polyglycerol monooleate having an average degree of polymerization of glycerol of 6 that was obtained in Example 9, and Moresco White P-70 (liquid paraffin, available from MORESCO Corporation) as an oil agent, in a ratio of 10 : 10 : 80 (weight ratio) was prepared. The HLB value of Sample 1 was 11.2.

Sample 2 (cosmetic composition) containing the polyglycerol monooleate having an average degree of polymerization of glycerol of 4 that was obtained in Example 8, the polyglycerol monooleate having an average degree of polymerization of glycerol of 10 that was obtained in Example 10, and Moresco White P-70 (liquid paraffin, available from MORESCO Corporation) as an oil agent, in a ratio of 10 : 10 : 80 (weight ratio) was prepared. The HLB value of Sample 2 was 12.2.

Sample 3 (cosmetic composition) containing the polyglycerol monooleate having an average degree of polymerization of glycerol of 4 that was obtained in Example 8, SY Glyster MO-7S (polyglycerol monooleate having an average degree of polymerization of glycerol of 10, available from Sakamoto Yakuhin Kogyo Co., Ltd.), and Moresco White P-70 (liquid paraffin, available from MORESCO Corporation) as an oil agent, in a ratio of 10 : 10 : 80 (weight ratio) was prepared. The HLB value of the sample 3 was 12.2.

A lipstick (trade name: Lipstick Y, available from Chifure Holdings Corporation) was applied to the arm of a subject, and Samples 1 to 3 were blended thereon and then washed off with water. Samples 1 to 3 were evaluated as follows.

[Makeup Removability]

○: Lipstick is removed cleanly.
×: Lipstick is not removed.

[Texture of Skin After Washing Off]

○: Refreshing
×: Sticky

As a result of the evaluation, in Samples 1 and 2, both [Makeup Removability] and [Texture of Skin After Washing Off] were "∘". On the other hand, in Sample 3, both [Makeup Removability] and [Texture of Skin After Washing Off] were "×". From these evaluations, it was found that the effects required for cosmetics, such as [Makeup Removability] and [Texture of Skin After Washing Off], are exhibited by blending the polyglycerol mono-fatty acid ester product of the present disclosure in a cosmetic composition.

To summarize the above, configurations and variations of the present disclosure are additionally described below.

[1] A polyglycerol mono-fatty acid ester product, in which

a fatty acid has from 4 to 25 carbon atoms,
a peak intensity ratio represented by Formula (X) below is 0.20 or more, and
a peak intensity ratio represented by Formula (Y) below is 0.46 or less.

$$\text{Formula (X)} = P2/P1$$

$$\text{Formula (Y)} = P3/P1$$

P1: a sum of peak intensities of a monoester form, a dehydration product of the monoester form, a diester form, a dehydration product of the diester form, a triester form, and a dehydration product of the triester form of a polyglycerol fatty acid ester, as well as peak intensities of polyglycerol and a dehydration product of the polyglycerol, when mass spectrometry of the polyglycerol mono-fatty acid ester product is performed using a time-of-flight mass spectrometer

P2: the peak intensity of the monoester form of the polyglycerol fatty acid ester when the mass spectrometry of the polyglycerol mono-fatty acid ester product is performed using the time-of-flight mass spectrometer

P3: a sum of the peak intensities of the polyglycerol and the dehydration product of the polyglycerol when the mass spectrometry of the polyglycerol mono-fatty acid ester product is performed using the time-of-flight mass spectrometer

[2] The polyglycerol mono-fatty acid ester product according to [1], in which the fatty acid has 6 or more, 8 or more, 10 or more, 12 or more, 14 or more, 15 or more, 16 or more, or 18 or more carbon atoms; and/or 22 or less, 20 or less, 18 or less, 16 or less, 14 or less, 12 or less, 10 or less, or 8 or less carbon atoms.

[3] The polyglycerol mono-fatty acid ester product according to [1] or [2], in which the fatty acid has from 4 to 14, from 15 to 25, from 8 to 14, or from 16 to 18 carbon atoms.

[4] The polyglycerol mono-fatty acid ester product according to any one of [1] to [3], in which the peak intensity ratio represented by Formula (X) is 0.21 or more, 0.23 or more, 0.24 or more, 0.25 or more, 0.26 or more, 0.28 or more, 0.3 or more, 0.31 or more, 0.33 or more, 0.35 or more, 0.37 or more, 0.40 or more, 0.44 or more, 0.48 or more, or 0.52 or more; and/or 1 or less, 0.9 or less, 0.8 or less, 0.75 or less, 0.7 or less, 0.65 or less, or 0.6 or less.

[5] The polyglycerol mono-fatty acid ester product according to any one of [1] to [4], in which the peak intensity ratio represented by Formula (Y) is 0.45 or less, 0.44 or less, 0.42 or less, 0.39 or less, 0.37 or less, 0.35 or less, 0.33 or less, 0.32 or less, or 0.30 or less; and/or 0.01 or more, 0.05 or more, or 0.10 or more.

[6] The polyglycerol mono-fatty acid ester product according to any one of [1] to [5], in which an average degree of polymerization of glycerol is 3 or more, 4 or more, 5 or more, 6 or more, 7 or more, 8 or more, or 9 or more; and/or 35 or less, 30 or less, 25 or less, 20 or less, 18 or less, 16 or less, 14 or less, 12 or less, 10 or less, 8 or less, 6 or less, or 4 or less.

[7] The polyglycerol mono-fatty acid ester product according to any one of [1] to [6], in which the average degree of polymerization of glycerol is from 2 to 40, from 3 to 20, or from 4 to 10.

[8] The polyglycerol mono-fatty acid ester product according to any one of [1] to [7], in which the fatty acid is a linear fatty acid or a branched fatty acid.

[9] The polyglycerol mono-fatty acid ester product according to any one of [1] to [8], in which the fatty acid is a saturated fatty acid or an unsaturated fatty acid.

[10] The polyglycerol mono-fatty acid ester product according to any one of [1] to [9], in which the fatty acid is at least one selected from the group consisting of caproic acid ($C_6$, saturated fatty acid), caprylic acid ($C_8$, saturated fatty acid), pelargonic acid ($C_9$, saturated fatty acid), 2-ethylhexanoic acid ($C_8$, branched fatty acid), capric acid ($C_{10}$, saturated fatty acid), lauric acid ($C_{12}$, saturated fatty acid), isotridecanoic acid ($C_{13}$, branched fatty acid), myristic acid ($C_{14}$, saturated fatty acid), pentadecanoic acid ($C_{15}$, saturated fatty acid), palmitic acid ($C_{16}$, saturated fatty acid), palmitoleic acid ($C_{16}$, unsaturated fatty acid), stearic acid ($C_{18}$, saturated fatty acid), isostearic acid ($C_{18}$, branched fatty acid), oleic acid ($C_{18}$, unsaturated fatty acid), linoleic acid ($C_{18}$, unsaturated fatty acid), ricinoleic acid ($C_{18}$, unsaturated fatty acid), hydroxystearic acid ($C_{18}$, substituted fatty acid), arachidic acid ($C_{20}$, saturated fatty acid), behenic acid ($C_{22}$, saturated fatty acid), erucic acid ($C_{22}$, unsaturated fatty acid), and nervonic acid ($C_{24}$, unsaturated fatty acid).

[11] The polyglycerol mono-fatty acid ester product according to any one of [1] to [10], in which an area ratio of peaks corresponding to the monoester form, the dehydration product of the monoester form, the polyester form, and the dehydration product of the polyester form of the polyglycerol fatty acid ester, as well as peaks corresponding to the polyglycerol and the dehydration product of the polyglycerol in high performance liquid chromatography (HPLC) analysis is 50% or more, 60% or more, 80% or more, or 90% or more.

[12] The polyglycerol mono-fatty acid ester product according to any one of [1] to [11], in which the area ratio of peaks corresponding to the monoester form, the dehydration product of the monoester form, the polyester form, and the dehydration product of the polyester form of the polyglycerol fatty acid ester in high performance liquid chromatography (HPLC) analysis is 5% or more, 10% or more, or 20% or more.

[13] The polyglycerol mono-fatty acid ester product according to any one of [1] to [12], in which the area ratio of peaks corresponding to the polyglycerol and the dehydration product of the polyglycerol in high performance liquid chromatography (HPLC) analysis is 80% or less, 70% or less, or 60% or less.

[14] The polyglycerol mono-fatty acid ester product according to any one of [1] to [13] that is a reaction product of glycidol and a fatty acid, an esterified form of polyglycerol and a fatty acid, or a transesterified form of polyglycerol and a fatty acid ester.

[15] A cosmetic composition containing the polyglycerol mono-fatty acid ester product described in any one of [1] to [14].

[16] The cosmetic composition according to [15], in which a content of the polyglycerol mono-fatty acid ester product is from 0.01 to 80 wt.%, from 0.1 to 50 wt.%, from 0.1 to 30 wt.%, or from 0.5 to 20 wt.%.

[17] The cosmetic composition according to [15] or [16], in which a HLB value of the contained polyglycerol mono-fatty acid ester product is from 9 to 13 or from 11 to 12.5.

[18] A method for producing a polyglycerol mono-fatty acid ester product, the method including carrying out an addition polymerization reaction of glycidol with a fatty acid.

[19] The method for producing a polyglycerol mono-fatty acid ester product according to [18], in which the addition polymerization reaction is carried out in the presence of an acidic catalyst.

[20] The method for producing a polyglycerol mono-fatty acid ester product according to [18] or [19], in which the acidic catalyst is at least one selected from the group consisting of ascorbic acid, acetic acid, formic acid, citric acid, succinic acid, adipic acid, and a phosphoric acid-based acidic catalyst.

[21] The method for producing a polyglycerol mono-fatty acid ester product according to [20], in which the phosphoric acid-based acidic catalyst is a phosphoric acid or an acidic phosphate ester.

[22] The method for producing a polyglycerol mono-fatty acid ester product according to any one of [18] to [21], in which the addition polymerization reaction is carried out by continuously or intermittently supplying glycidol to the fatty acid.

[23] The method for producing a polyglycerol mono-fatty acid ester product according to any one of [18] to [21], in which the addition polymerization reaction is carried out by, while continuously or intermittently supplying glycidol to the fatty acid, continuously or intermittently supplying the acidic catalyst.

**Claims**

1. A polyglycerol mono-fatty acid ester product, wherein

    a fatty acid has 4 to 25 carbon atoms,
    a peak intensity ratio represented by Formula (X) is 0.20 or more, and
    a peak intensity ratio represented by Formula (Y) is 0.46 or less,

$$\text{Formula (X)} = P2/P1$$

$$\text{Formula (Y)} = P3/P1$$

   P1: a sum of peak intensities of a monoester form, a dehydration product of the monoester form, a diester form, a dehydration product of the diester form, a triester form, and a dehydration product of the triester form of a polyglycerol fatty acid ester, as well as peak intensities of polyglycerol and a dehydration product of the polyglycerol, when mass spectrometry of the polyglycerol mono-fatty acid ester product is performed using a time-of-flight mass spectrometer,
   P2: the peak intensity of the monoester form of the polyglycerol fatty acid ester when the mass spectrometry of the polyglycerol mono-fatty acid ester product is performed using the time-of-flight mass spectrometer,
   P3: a sum of the peak intensities of the polyglycerol and the dehydration product of the polyglycerol when the mass spectrometry of the polyglycerol mono-fatty acid ester product is performed using the time-of-flight mass spectrometer.

2. The polyglycerol mono-fatty acid ester product according to claim 1, wherein the fatty acid has from 15 to 25 carbon atoms.

3. The polyglycerol mono-fatty acid ester product according to claim 1, wherein the fatty acid has from 4 to 14 carbon atoms.

4. The polyglycerol mono-fatty acid ester product according to claim 2, wherein the fatty acid has from 16 to 18 carbon atoms.

5. The polyglycerol mono-fatty acid ester product according to claim 3, wherein the fatty acid has from 8 to 14 carbon atoms.

6. The polyglycerol mono-fatty acid ester product according to claim 2 or 3, wherein an average degree of polymerization of glycerol is from 2 to 20.

7. The polyglycerol mono-fatty acid ester product according to claim 2 or 3, wherein the fatty acid is a linear fatty acid.

8. The polyglycerol mono-fatty acid ester product according to claim 2 or 3, wherein the fatty acid is an unsaturated fatty acid.

9. The polyglycerol mono-fatty acid ester product according to claim 2 or 3, wherein the fatty acid is a branched fatty acid.

10. The polyglycerol mono-fatty acid ester product according to claim 1, wherein the polyglycerol mono-fatty acid ester product is a reaction product of glycidol and a fatty acid.

11. A cosmetic composition comprising the polyglycerol mono-fatty acid ester product described in claim 1.

12. A method for producing a polyglycerol mono-fatty acid ester product, the method comprising carrying out an addition polymerization reaction of glycidol with a fatty acid.

13. The method for producing a polyglycerol mono-fatty acid ester product according to claim 12, wherein the addition polymerization reaction is carried out by continuously or intermittently supplying glycidol to the fatty acid.

14. The method for producing a polyglycerol mono-fatty acid ester product according to claim 12, wherein the addition polymerization reaction is carried out by, while continuously or intermittently supplying glycidol to the fatty acid, continuously or intermittently supplying an acidic catalyst.

15. The method for producing a polyglycerol mono-fatty acid ester product according to claim 13 or 14, wherein the acidic catalyst is a phosphoric acid or an acidic phosphate ester.

FIG. 1

FIG. 2

## INTERNATIONAL SEARCH REPORT

| International application No. |
|---|
| **PCT/JP2024/019098** |

| A. | CLASSIFICATION OF SUBJECT MATTER |
|---|---|

*C07C 69/30*(2006.01)i; *A61K 8/86*(2006.01)i; *A61Q 5/00*(2006.01)i; *A61Q 19/00*(2006.01)i; *C07B 61/00*(2006.01)i; *C07C 67/26*(2006.01)i; *C07C 69/58*(2006.01)i; *C08G 65/22*(2006.01)i; *C11C 3/00*(2006.01)i
FI: C07C69/30; C11C3/00; A61K8/86; A61Q19/00; A61Q5/00; C07B61/00 300; C08G65/22; C07C67/26; C07C69/58

According to International Patent Classification (IPC) or to both national classification and IPC

| B. | FIELDS SEARCHED |
|---|---|

Minimum documentation searched (classification system followed by classification symbols)

C07B31/00-63/04, C07C1/00-409/44

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2015-119648 A (NISSHIN OILLIO GROUP LTD.) 02 July 2015 (2015-07-02) | 1-10 |
| | paragraphs [0001], [0009], examples | |
| Y | paragraphs [0001], [0009], examples | 1-11 |
| X | JP 2006-232714 A (RIKEN VITAMIN CO., LTD.) 07 September 2006 (2006-09-07) | 1-10 |
| | paragraphs [0001], [0011], examples | |
| Y | paragraphs [0001], [0011], examples | 1-11 |
| X | CN 111233637 A (SOUTH CHINA INSTITUTE OF COLLABORATIVE INNOVATION) 05 June 2020 (2020-06-05) | 1, 3, 5-7, 10 |
| | claim 1, table 4 | |
| Y | claim 1, table 4 | 1-11 |
| X | JP 2001-139679 A (DAICEL CHEM. IND. LTD.) 22 May 2001 (2001-05-22) | 12-15 |
| | claims, examples | |
| Y | paragraph [0001] | 1-11 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **05 July 2024** | **23 July 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

| International application No. |
|---|
| **PCT/JP2024/019098** |

### C. DOCUMENTS CONSIDERED TO BE RELEVANT

| Category* | Citation of document, with indication, where appropriate, of the relevant passages | Relevant to claim No. |
|---|---|---|
| X | JP 11-222521 A (DAICEL CHEM. IND. LTD.) 17 August 1999 (1999-08-17) claims, examples | 12-15 |
| Y | paragraph [0001] | 1-11 |
| Y | WO 2004/048304 A1 (DAICEL CHEM. IND. LTD.) 10 June 2004 (2004-06-10) page 1, lines 6-12 | 11 |
| X | JP 09-249615 A (DAICEL CHEM. IND. LTD.) 22 September 1997 (1997-09-22) claims, examples | 12-15 |
| Y | | 1-11 |
| X | JP 2010-260814 A (RIKEN VITAMIN CO., LTD.) 18 November 2010 (2010-11-18) paragraph [0024] | 12-15 |
| Y | | 1-11 |
| A | JP 2022-176113 A (NOF CORPORATION) 25 November 2022 (2022-11-25) | 1-15 |

Form PCT/ISA/210 (second sheet) (July 2022)

## INTERNATIONAL SEARCH REPORT

International application No.

**PCT/JP2024/019098**

| Box No. III | Observations where unity of invention is lacking (Continuation of item 3 of first sheet) |
| --- | --- |

This International Searching Authority found multiple inventions in this international application, as follows:

(Invention 1) Claims 1-11

Documents 1-3 respectively disclose the polyglyceryl monofatty acid ester product of the invention in claim 1 of the present application. Claims 1-10 lack novelty in light of documents 1 and 2 and thus do not have a special technical feature. Claims 1-10 and claim 11 dependent on claim 1 therefore are classified as invention 1.

(Invention 2) Claims 12-15

Claims 12-15 cannot be said to share a same or corresponding special technical feature with claim 11 classified as invention 1.

Furthermore, claims 12-15 are not substantially identical to or similarly closely related to any of the claims classified as invention 1.

Claims 12-15 therefore cannot be classified as invention 1.

Claim 12 has the special technical feature of a "method for producing a polyglyceryl monofatty acid ester product, comprising an addition polymerization reaction step of a glycidol and a fatty acid", and thus is classified as invention 2.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
Information on patent family members

| International application No. |
| --- |
| **PCT/JP2024/019098** |

| Patent document cited in search report | | | Publication date (day/month/year) | Patent family member(s) | | | Publication date (day/month/year) |
| --- | --- | --- | --- | --- | --- | --- | --- |
| JP | 2015-119648 | A | 02 July 2015 | (Family: none) | | | |
| JP | 2006-232714 | A | 07 September 2006 | (Family: none) | | | |
| CN | 111233637 | A | 05 June 2020 | (Family: none) | | | |
| JP | 2001-139679 | A | 22 May 2001 | (Family: none) | | | |
| JP | 11-222521 | A | 17 August 1999 | (Family: none) | | | |
| WO | 2004/048304 | A1 | 10 June 2004 | US | 2006/0258823 | A1 | |
| | | | | paragraph [0001] | | | |
| | | | | EP | 1568677 | A1 | |
| | | | | AU | 2003302419 | A | |
| | | | | KR | 10-2005-0084009 | A | |
| | | | | CN | 1717381 | A | |
| | | | | TW | 200426136 | A | |
| JP | 09-249615 | A | 22 September 1997 | US | 6278008 | B1 | |
| | | | | claims, examples | | | |
| | | | | EP | 758641 | A1 | |
| JP | 2010-260814 | A | 18 November 2010 | (Family: none) | | | |
| JP | 2022-176113 | A | 25 November 2022 | CN | 115340456 | A | |
| | | | | KR | 10-2022-0154614 | A | |

Form PCT/ISA/210 (patent family annex) (July 2022)

REFERENCES CITED IN THE DESCRIPTION

**Patent documents cited in the description**

- JP 8109153 A
- JP 9117258 A
- JP 9216813 A
- JP 9272893 A
- JP 2006111539 A
- WO 2004048304 A
- JP 2001139679 A
- JP 2017071586 A